(19) Europäisches Patentamt
European Patent Office
Office européen des brevets

(11) EP 3 360 536 A1

(12) # EUROPEAN PATENT APPLICATION
published in accordance with Art. 153(4) EPC

(43) Date of publication:
**15.08.2018 Bulletin 2018/33**

(21) Application number: **16853247.1**

(22) Date of filing: **29.09.2016**

(51) Int Cl.:
***A61K 8/81*** *(2006.01)* ***A61K 8/06*** *(2006.01)*
***A61K 8/37*** *(2006.01)* ***A61K 8/86*** *(2006.01)*
***A61Q 17/04*** *(2006.01)*

(86) International application number:
**PCT/JP2016/004404**

(87) International publication number:
**WO 2017/061090 (13.04.2017 Gazette 2017/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.10.2015 JP 2015201572**
**27.10.2015 PCT/JP2015/005404**

(71) Applicant: **Dow Corning Toray Co., Ltd.**
**Tokyo 100-0004 (JP)**

(72) Inventors:
- **SUGIURA, Tsunehito**
**Iichihara-shi**
**Chiba 299-0108 (JP)**
- **KANZAKI, Yasue**
**Iichihara-shi**
**Chiba 299-0108 (JP)**
- **HORI, Seiji**
**Iichihara-shi**
**Chiba 299-0108 (JP)**

(74) Representative: **Murgitroyd & Company**
**Scotland House**
**165-169 Scotland Street**
**Glasgow G5 8PL (GB)**

(54) **FILM FORMING AGENT, AND COSMETIC COMPOSITION AND COSMETIC USING SAME**

(57) A cosmetic composition comprising at least: (A) 100 parts by weight of at least one vinyl polymer having at least one carbosiloxane dendrimer group in a side chain; (B) from 0.1 to 30 parts by weight of a surfactant; and (X) water; wherein an emulsified particle comprising at least the component (A) and the component (B) is in an aqueous phase; and a weight average molecular weight of the component (A) is at least 80000. By blending a vinyl polymer emulsion having a carbosiloxane dendrimer structure in an aqueous phase according to the present invention, a cosmetic that exhibits excellent softness, texture during usage and smoothness of finish, waterproofness, and makeup retaining properties can be provided.

EP 3 360 536 A1

## Description

[TECHNICAL FIELD]

The present invention relates to a film forming agent, a cosmetic composition using the same, and a cosmetic containing the same.

[BACKGROUND ART]

In cosmetic materials, typically, water-in-oil emulsions can impart physical properties that can enhance waterproofness and/or long-lasting characteristics, and thus such water-in-oil emulsions have been used in a wide variety of skin care products, such as sunscreen products. A technique, in which a vinyl polymer having a carbosiloxane dendrimer structure in its side chain is used to form a coating film has been developed. By applying this to a cosmetic, a cosmetic coating film is formed to enhance makeup retaining properties, such as waterproofness, sebum resistance, and rub resistance. Therefore, taking advantage of ability to blend a large amount of an oil-soluble film forming agent, water-in-oil skin cosmetic materials containing, in its oil phase, a vinyl polymer having a carbosiloxane dendrimer structure in its side chain have been developed. (Patent Document 1, Patent Document 2, and Patent Document 3)

However, the water-in-oil skin cosmetic material has problems in that the water-in-oil skin cosmetic material cannot contain a large amount of an oil-soluble film forming agent because of problems due to blendability with other cosmetic components. The water-in-oil skin cosmetic material also has problems of texture during usage such as greasiness and stickiness which are characteristics of the water-in-oil emulsion. On the other hand, although oil-in-water emulsion can impart fresh texture during usage, a sufficient amount of a film forming agent cannot be used, and there are problems such as poor waterproofness and poor long-lasting characteristics. Furthermore, for applications in coating, a technique in which copolymerization is performed in an aqueous phase has been also developed. (Patent Document 4)

[PRIOR ART DOCUMENTS]

[Patent Documents]

Patent Document 1: JP 07-309714 A
Patent Document 2: JP 2007-320960 A
Patent Document 3: JP 2014-40512 A
Patent Document 4: JP 2001-19242 A

[SUMMARY OF THE INVENTION]

[Problems to be Solved by the Invention]

In the present invention, a high molecular weight vinyl polymer can be formed in an oil phase of an emulsion particle by using a vinyl polymer emulsion having a carbosiloxane dendrimer structure in its side chain in an aqueous phase. Thus, an object is to provide a film forming agent that exhibits excellent texture during usage and smoothness of finish, waterproofness and rub resistance, sebum resistance, makeup retaining properties, softness, and integrity as well as that overcomes technical problems caused by an aqueous phase polymerization when used for application in cosmetics. Furthermore, besides the application in cosmetics, the film forming agent can be used in general film applications that requires effect of water repellency (waterproofness), effect of oil repellency (oil resistance), rub resistance, softness, and integrity.

[MEANS FOR SOLVING THE PROBLEMS]

A cosmetic composition including at least:

(A) 100 parts by weight of at least one vinyl polymer having at least one carbosiloxane dendrimer structure in a side chain;
(B) from 0.1 to 30 parts by weight of a surfactant; and
(X) water;
wherein an emulsified particle including at least the component (A) and the component (B) is in an aqueous phase;

and a weight average molecular weight of the component (A) is at least 80000.

[EFFECTS OF THE INVENTION]

The film forming agent and the cosmetic composition using the same of an embodiment of the present invention make the polymer designing easier and exhibit excellent texture during usage and smoothness of finish, waterproofness, sebum resistance, makeup retaining properties and rub resistance, softness, integrity, and adhesion to hair. Furthermore, in particular, the compounded amount of the carbosiloxane dendrimer can be easily adjusted, the emulsified particle size can be finely adjusted, and high stability of the emulsion can be achieved.

[BRIEF DESCRIPTION OF THE DRAWINGS]

FIG. 1 is a photograph taken after mascara containing a film forming agent (A) and a Control (sample containing no film forming agent (A)) for comparison are coated on a PMMA plate that simulates skin and then rubbed from above by using a felt material in "Long-lasting characteristics (rub resistance) evaluation of mascara formulation" according to Cosmetic Material Evaluation Example 2.

FIG. 2 is a recorded curve that evaluates the long-lasting characteristics by observing a change in color tone $\Delta E$ when a lipstick containing a film forming agent (A) and a Control (sample containing no film forming agent (A)) for comparison are coated on a PMMA plate that simulates skin and then rubbed from above by using a felt material according to Cosmetic Material Evaluation Example 3. (In FIG. 2, the horizontal axis indicates the number of times of rubbing with a probe, and the vertical axis indicates $\Delta E$.)

[MODE FOR CARRYING OUT THE INVENTION]

At least one vinyl polymer having at least one carbosiloxane dendrimer structure in its side chain of the component (A) of an embodiment of the present invention is formed by copolymerization of a component (a1) unsaturated monomer and a component (a2) unsaturated monomer that is different from the component (a1) as a carbosiloxane dendrimer structure. The component (a1) is preferably an unsaturated monomer represented by Formula (1) below.

(a1) An unsaturated monomer having a carbosiloxane dendrimer structure is represented by Formula (1) below.

[Formula 1]

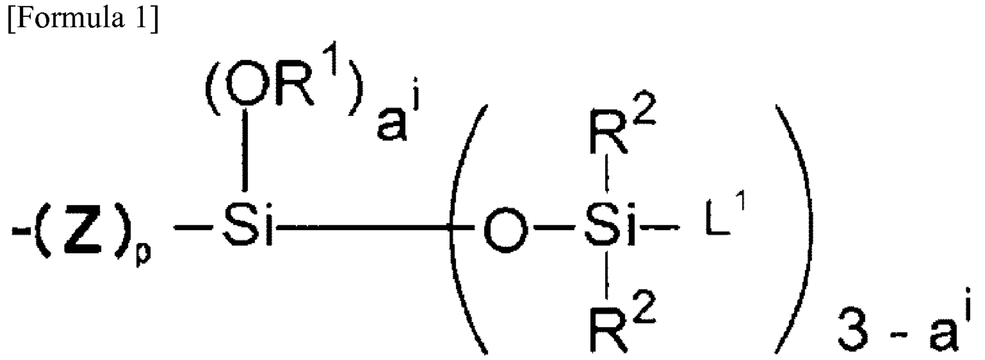

(1)

wherein

Z is a divalent organic group,
p is 0 or 1,
$R^1$ and $R^2$ are each independently an alkyl group having from 1 to 10 carbons, an aryl group, or an aralkyl group, and
$L^1$ is a silylalkyl group represented by Formula (2) below in the case where i = 1.

[Formula 2]

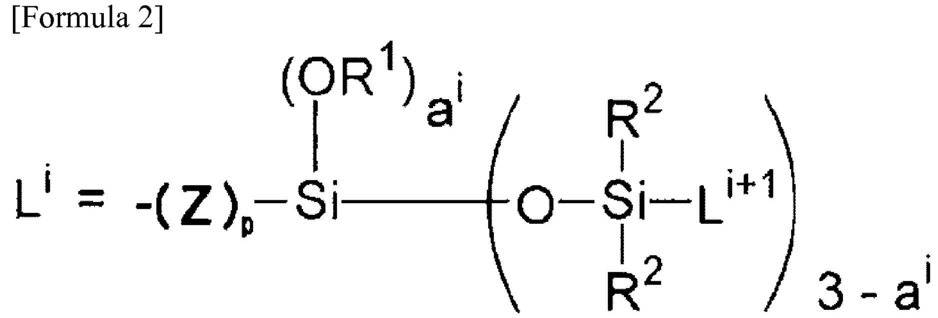

(2)

wherein

Z and p are the same as those described above,
$R^1$ and $R^2$ are the same as those described above,
i is an integer of from 1 to 10 representing the total number of generations of the silylalkyl group,
$L^{i+1}$ is a group selected from the group consisting of a hydrogen atom, an alkyl group having from 1 to 10 carbons, an aryl group, an aralkyl group, and the silyl alkyl group; however, in the case of i = c (c is an integer of from 1 to 10 representing the generations of the silylalkyl group), $L^{i+1}$ is a hydrogen atom, an alkyl group having from 1 to 10 carbons, an aryl group, or an aralkyl group, and in the case of i < c, $L^{i+1}$ is the silylalkyl group, and $a^i$ is an integer of 0 to 3.

Note that the carbosiloxane dendrimer structure is a chemical structure that is highly and radially branched from one silicon atom, and i representing the total number of generations of the silylalkyl group indicates the degree of branching. For example, in a case where the total number of generations i is 1 and $L^{i+1}$ is, for example, a methyl group, the carbosiloxane dendrimer structure has the following structure.

[Formula 3]

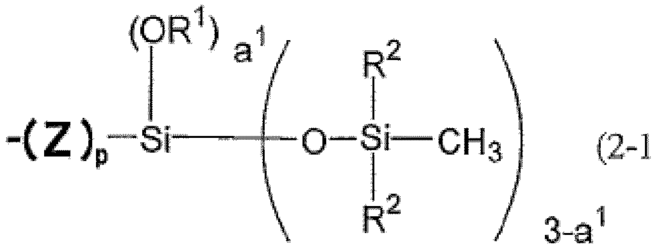

(2-1)

wherein Z, p, $R^1$, and $R^2$ are the same as those described above, and $a^1$ is an integer of from 0 to 3.

Similarly, in a case where the generation i is 2 and $L^{i+1}$ is, for example, a methyl group, the carbosiloxane dendrimer structure has the following structure however, p = 1).

[Formula 4]

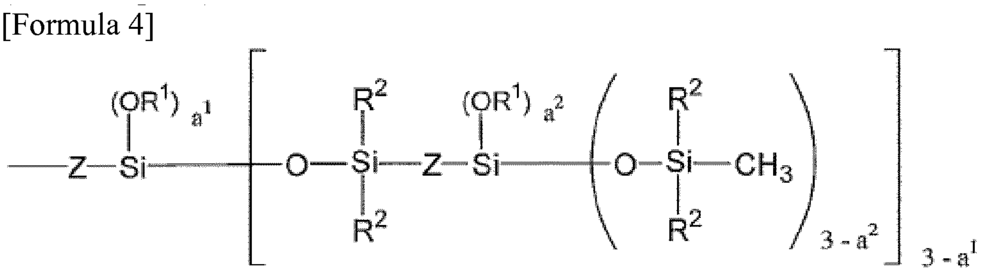

(2-2)

wherein Z, $R^1$, and $R^2$ are the same as those described above, and $a^1$ and $a^2$ are each an integer of from 0 to 3.

The particularly preferable carbosiloxane dendrimer structure is the following carbosiloxane dendrimer structure.

[Formula 5]

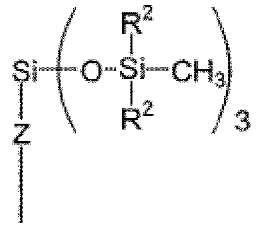

wherein Z and $R^2$ are the same as those described above.

[Formula 6]

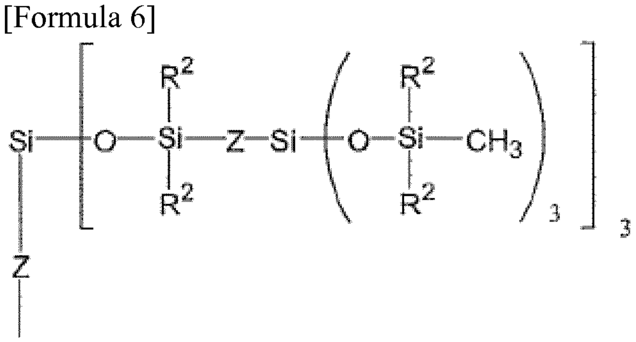

wherein Z and $R^2$ are the same as those described above.

As long as the component (a2) unsaturated monomer constituting the (A) carbosiloxane dendrimer structure is an unsaturated monomer that has a radically polymerizable vinyl group that is different from the component (a1), the type thereof and the like are not particularly limited. Examples of such a vinyl-based monomer include monomers that are starting materials of organic resins which are typically called vinyl-based resins, and specific examples thereof include lower alkyl(meth)acrylate such as methyl(meth)acrylate, ethyl(meth)acrylate, n-propyl(meth)acrylate, and isopropyl(meth)acrylate; glycidyl(meth)acrylate; higher (meth)acrylates such as n-butyl(meth)acrylate, isobutyl(meth)acrylate, tert-butyl(meth)acrylate, n-hexyl(meth)acrylate, cyclohexyl(meth)acrylate, 2-ethylhexyl(meth)acrylate, octyl(meth)acrylate, lauryl(meth)acrylate, and stearyl(meth)acrylate; lower fatty acid vinyl esters such as vinyl acetate and vinyl propionate; higher fatty acid esters such as vinyl butyrate, vinyl caproate, vinyl 2-ethylhexanoate, vinyl laurate, and vinyl stearate; aromatic vinyl-based monomers such as styrene, vinyltoluene, benzyl(meth)acrylate, phenoxyethyl(meth)acrylate, and vinylpyrrolidone; amide group-containing vinyl-based monomers such as (meth)acrylamide, N-methylol(meth)acrylamide, N-methoxymethyl(meth)acrylamide, isobutoxymethoxy(meth)acrylamide, and N,N-dimethyl(meth)acrylamide; hydroxy group-containing vinyl-based monomers such as 2-hydroxyethyl(meth)acrylate, 2-hydroxybutyl(meth)acrylate, and 2-hydroxypropyl(meth)acrylate; fluorine-containing vinyl-based monomers such as trifluoropropyl(meth)acrylate, perfluorobutylethyl(meth)acrylate, and perfluorooctylethyl(meth)acrylate; epoxy group-containing vinyl-based monomers such as glycidyl(meth)acrylate and 3,4-epoxycyclohexylmethyl(meth)acrylate; carboxylic acid-containing vinyl-based monomers such as (meth)acrylic acid, itaconic acid, crotonic acid, fumaric acid, and maleic acid; ether bond-containing vinyl-based monomers such as tetrahydrofurfuryl(meth)acrylate, butoxyethyl(meth)acrylate, ethoxydiethylene glycol (meth)acrylate, polyethylene glycol (meth)acrylate, polypropylene glycol mono(meth)acrylate, hydroxybutyl vinyl ether, cetyl vinyl ether, and 2-ethylhexyl vinyl ether; unsaturated group-containing silicone compounds such as (meth)acryloxypropyltrimethoxysilane, (branched or linear) polydimethylsiloxane having a (meth)acryl group at one end, and polydimethylsiloxane having a styryl group at one end; butadiene; vinyl chloride; vinylidene chloride; (meth)acrylonitrile; dibutyl fumarate; maleic anhydride; dodecyl succinic anhydride; (meth)acryl glycidyl ether; alkali metal salts, ammonium salts, and organic amine salts of radically polymerizable unsaturated carboxylic acids such as (meth)acrylic acid, itaconic acid, crotonic acid, fumaric acid, and maleic acid; radically polymerizable unsaturated monomers each having a sulfonic acid group, such as styrenesulfonic acid, and alkali metal salts, ammonium salts, and organic amine salts thereof; quaternary ammonium salts derived from (meth)acrylic acid, such as 2-hydroxy-3-methacryloxypropyltrimethylammonium chloride; and methacrylic acid esters of alcohols each having a tertiary amine group, such as methacrylic acid diethylamine ester, and quaternary ammonium salts thereof.

Furthermore, polyfunctional vinyl-based monomers can be also used, and examples thereof include trimethylolpropane tri(meth)acrylate, pentaerythritol tri(meth)acrylate, ethylene glycol di(meth)acrylate, tetraethylene glycol di(meth)acrylate, polyethylene glycol di(meth)acrylate, 1,4-butanediol di(meth)acrylate, 1,6-hexanediol di(meth)acrylate, neopentyl glycol di(meth)acrylate, trimethylolpropane trioxyethyl(meth)acrylate, tris(2-hydroxyethyl)isocyanurate

di(meth)acrylate, tris(2-hydroxyethyl)isocyanurate tri(meth)acrylate, di(meth)acrylate of an ethylene oxide or propylene oxide adduct of bisphenol A as a diol, di(meth)acrylate of an ethylene oxide or propylene oxide adduct of hydrogenated bisphenol A as a diol, (meth)acryloyl group-containing monomers such as triethylene glycol divinyl ether, unsaturated group-containing silicone compounds such as polydimethylsiloxane having both ends capped with styryl groups and polydimethylsiloxane having both ends capped with methacryloxypropyl, and the like.

Furthermore, an organosilicon compound having a vinyl-based radically polymerizable unsaturated group and a hydrolyzable group can be used. This case is preferable because film strength is larger and long-lasting characteristics of water repellency is enhanced. Note that examples of the radically polymerizable group include (meth)acryloxy group-containing organic groups, (meth)acrylamide group-containing organic groups, styryl group-containing organic groups, alkenyl groups having from 2 to 10 carbons, a vinyloxy group, an allyloxy group, and the like.

Among these, the particularly preferable unsaturated monomer of component (a2) is an acrylate-based monomer or a methacrylate-based monomer. Among these, particularly preferable unsaturated monomers are acryl group- or methacryl group-containing organic groups represented by general formulas below:

[Formula 7]

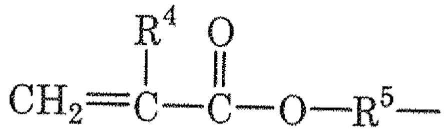

wherein $R^4$ is a hydrogen atom or a methyl group, and $R^5$ is an alkylene group having from 1 to 10 carbons; or

[Formula 8]

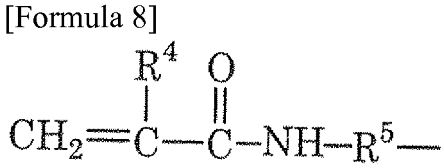

wherein $R^4$ and $R^5$ are the same as those described above.

The vinyl polymer containing the dendrimer structure used in an embodiment of the present invention is formed by copolymerizing the component (a1) and the component (a2), and the weight ratio (a1):(a2) at the time of the copolymerization is preferably from 10:90 to 90:10, more preferably from 20:80 to 85:15, and even more preferably from 30:70 to 60:40. Furthermore, the amount of the component (a1) is preferably from 45 wt.% to 60 wt.% in all the monomer units.

In the production method of the cosmetic composition used in an embodiment of the present invention, the cosmetic composition is obtained by copolymerizing the following components (A) and (B) in an aqueous phase:

(A) (a1) unsaturated monomer having a carbosiloxane dendrimer structure having a radically polymerizable organic group, and
(a2) a monomer having a radically polymerizable vinyl group, the monomer being different from the component (a1); and
(B) a surfactant
(however, the amount of the component (B) is in a range of 0.1 to 30 parts by weight per 100 parts by weight of the component (A)). As the weight ratio of the component (B) to the component (A), the weight of the component (B) is preferably from 0.1 to 10 parts by weight per 100 parts by weight of the component (A).

Regarding the polymerization of the carbosiloxane dendrimer, production can be performed in accordance with production methods described in, for example, JP 11-1530 A, JP 2000-63225 A, JP 2001-192424 A, JP 2014-40512 A, and the like.

The component (A) of an embodiment of the present invention is a water-dispersible vinyl copolymer and is obtained by adding from 0.01 to 20 parts by weight of a radical polymerization initiator to 100 parts by weight of the mixture of the component (a1) and the component (a2) to prepare an emulsion dispersion (cosmetic composition) in an aqueous medium containing the component (B) and then subjecting this to emulsion polymerization (copolymerization). As a result, even in oil-in-water cosmetics and water-in-oil cosmetics, an emulsion particle has a hydrophilic group in its periphery.

Furthermore, at this time, the component (a1), the component (a2), and the radical polymerization initiator may be emulsion-dispersed together, or the addition may be performed after the radical polymerization initiator is emulsion-dispersed in advance. Note that as long as the radical polymerization initiator is a radical polymerization initiator that is typically used in emulsion polymerization of a vinyl polymer, it is not particularly limited. Examples thereof include water-soluble peroxides, including inorganic peroxides such as potassium persulfate, sodium persulfate, and ammonium persulfate; and organic peroxides such as t-butylperoxy maleic acid, succinic acid peroxide, and t-butyl hydroperoxide. When an oil-soluble radical initiator is used, the oil-soluble radical initiator may be mixed with other components after the oil-soluble radical initiator is emulsified. The compounded amount of the radical polymerization initiator is in a range from 0.01 to 20 parts by weight, and preferably in a range from 0.1 to 10 parts by weight, per 100 parts by weight total of the component (a1) and the component (a2).

Furthermore, the emulsion dispersion is prepared by using ordinary emulsifying apparatus such as a colloid mill and/or a homogenizer. The polymerization is performed after pre-heating the reaction system to 50 to 90°C and performed for approximately 2 to 8 hours. At this time, the polymerization may be performed while the emulsion dispersion is added dropwise, or the polymerization may be performed after the emulsion dispersion is charged at once.

The number average molecular weight of the vinyl polymer having the carbosiloxane dendrimer structure of the component (A) used in an embodiment of the present invention is preferably from 3000 to 50000000, and more preferably from 5000 to 10000000, from the perspective of ease in blending as a cosmetic composition. Furthermore, from the perspectives of strength and performance of the film forming agent and ease in blending, the weight average molecular weight is at least 80000, more preferably 160000 or greater, 240000 or greater, and even more preferably 500000 or greater. Furthermore, the upper limit of the weight average molecular weight is 80000000 or less, preferably 40000000 or less, and even more preferably 10000000, or 8000000 or less.

The property thereof may be liquid, gum-like, paste-like, solid, or the like at room temperature; however, from the perspective of long-lasting characteristics of the obtained cosmetic film, solid is preferable. From the perspective of blendability, use in a form of a solution diluted with a solvent or a dispersion is preferable.

One or more types may be used as the component (A), and the amount of the component (A) is from 0.1 to 15 wt.%, and preferably from 0.5 to 10 wt.%, relative to the amount of the entire composition. In a case where the amount is less than 0.1 wt.%, it is difficult to fix makeup, and in a case where the amount is greater than 30 wt.%, unevenness in coating occurs, and smoothness tends to be poor.

Surfactant

To embody the invention of the present application, the component (B) surfactant is used depending on the application thereof. In particular, as the surfactant used for emulsification in a cosmetic, a component (B) ionic surfactant (anionic surfactant, cationic surfactant, amphoteric surfactant, semipolar surfactant) and a nonionic surfactant are mainly used. One type or a combination of two or more types of surfactants can be also used. Furthermore, for this component (B), a reactive surfactant having a radically polymerizable group can be also used. Furthermore, for a skin cosmetic, an anionic surfactant and/or a nonionic surfactant is preferably used. Furthermore, for a hair cosmetic, a cationic surfactant and/or a nonionic surfactant can be used.

Examples of the anionic surfactant include saturated or unsaturated fatty acid salts (e.g. sodium laurate, sodium stearate, sodium oleate, sodium linolenate, and the like), alkylsulfuric acid salts, alkylbenzene sulfonic acids (e.g. hexylbenzenesulfonic acid, toctylbenzenesulfonic acid, dodecylbenzenesulfonic acid, and the like) and salts thereof, polyoxyalkylene alkyl ether sulfate salts, polyoxyalkylene alkenyl ether sulfate salts, polyoxyethylene alkylsulfate salts, alkyl sulfosuccinate salts, alkyl polyoxyalkylene sulfosuccinate salts, polyoxyalkylene alkylphenyl ether sulfate salts, alkane sulfonate salts, octyltrimethylammonium hydroxide, dodecyltrimethylammonium hydroxide, alkyl sulfonates, polyoxyethylene alkylphenyl ether sulfate salts, polyoxyalkylene alkyl ether acetate salts, alkyl phosphate salts, polyoxyalkylene alkyl ether phosphate salts, acylglutamate salts, $\alpha$-acylsulfonate salts, alkylsulfonate salts, alkylallylsulfonate salts, $\alpha$-olefin sulfonate salts, alkylnaphthalene sulfonate salts, alkane sulfonate salts, alkyl or alkenylsulfate salts, alkylamide sulfate salts, alkyl- or alkenyl phosphate salts, alkylamide phosphate salts, alkyloylalkyl taurinate salts, N-acylamino acid salts, sulfosuccinate salts, alkyl ether carboxylate salts, amide ether carboxylate salts, $\alpha$-sulfofatty acid ester salts, alanine derivatives, glycine derivatives, arginine derivatives, and alkyl sulfoacetates.

From the perspective of low skin irritation, an anionic surfactant formed from phosphoric acid, carboxylic acid, or amino acid is preferable, and examples thereof include alkyl ether carboxylates, unsaturated fatty acid salts (e.g. sodium laurate, sodium stearate, sodium oleate, sodium linoleate, and the like), octyltrimethylammonium hydroxide, dodecyltrimethylammonium hydroxide, polyoxyalkylene alkyl ether acetate salts, alkyl phosphate salts, polyoxyalkylene alkyl ether phosphate salts, acylglutamate salts, alkyl- or alkenyl phosphate salts, alkylamide phosphate salts, alkyloylalkyl taurinate salts, N-acyl amino acid salts, alkyl ether carboxylate salts, amide ether carboxylate salts, alanine derivatives, glycine derivatives, arginine derivatives, alkyl ether carboxylate salts, polyoxyalkylene alkyl ether carboxylate salts, alkyl phosphate salts, polyoxyalkylene alkyl ether phosphate salts, N-acyl amino acid salts, alkaline earth metal salts, magnesium salts, alkanolamine salts, triethanolamine salts, and ammonium salts. Furthermore, an anionic surfactant formed from phosphoric acid is preferable.

The counterion of the hydrophilic group of the anionic surfactant is preferably $Na^+$, $K^+$, or $NH_4^+$.

Furthermore, the anionic surfactant is preferably an anionic surfactant having from 6 to 22 carbons, and more preferably from 8 to 18 carbons. In a case where the number of carbons is greater than the upper limit or lower than the lower limit, film formability may be insufficient or problems may occur during blending with other cosmetic composition when a cosmetic composition is formed. Specific examples of the preferable anionic surfactant include sodium lauryl phosphate and laureth-1 phosphate.

Examples of the nonionic surfactant include polyoxyalkylene ethers, polyoxyalkylene alkyl ethers, polyoxyalkylene fatty acid esters, polyoxyalkylene fatty acid diesters, polyoxyalkylene resinic acid esters, polyoxyalkylene (hydrogenated) castor oils, polyoxyalkylene alkyl phenols, polyoxyalkylene alkyl phenyl ethers, polyoxyalkylene phenyl phenyl ethers, polyoxyalkylene alkyl esters, polyoxyalkylene alkyl esters, sorbitan fatty acid esters, polyoxyalkylene sorbitan alkyl esters, polyoxyalkylene sorbitan fatty acid esters, polyoxyalkylene sorbitol fatty acid esters, polyoxyalkylene glyceric fatty acid esters, polyglycerol alkyl ethers, polyglycerol fatty acid esters, sucrose fatty acid esters, fatty acid alkanolamides, alkylglucosides, polyoxyalkylene fatty acid bisphenyl ethers, polypropylene glycols, diethylene glycol, polyoxyalkylene-modified silicones, polyglyceryl-modified silicones, glyceryl-modified silicones, saccharide-modified silicones, fluorine-based surfactants, polyoxyethylene-polyoxypropylene block polymers, and alkyl polyoxyethylene-polyoxypropylene block polymer ethers. In particular, polyoxyalkylene-modified silicones, polyglyceryl-modified silicones, glyceryl-modified silicones, and saccharide alcohol-modified silicones, which have an alkyl branch, linear-chain silicone branch, siloxane dendrimer branch, or the like as necessary, may be used simultaneously with the hydrophilic group.

In the present invention, the method of measuring the HLB value of the nonionic surfactant is a method in accordance with the actual measurement of the HLB value by an emulsion method described in "Handbook - cosmetic material/preparation ingredient - revised edition "Handbook -Cosmetics, Formulation raw materials -Revised", Nikko Chemicals Co., Ltd., revised edition published on February 1, 1977, pp. 854-855. The HLB value is a value indicating the balance between strengths of the hydrophobic group and the hydrophilic group constituting the surfactant, and typically, the HLB value of 1 to 8 indicates hydrophobicity, of greater than 8 but less than 10 indicates a property in between hydrophilic and hydrophobic, and of 10 or greater indicates hydrophilicity; however, this may be different depending on the structure of the surfactant.

The specific method of determining the HLB value of the (B) nonionic surfactant component include combining with sorbitan monostearate (NIKKOL SS-10, HLB 4.7) as a reference material of emulsifier, emulsifying a liquid paraffin (HLB 10.1) which is a target to be emulsified by only changing the proportion of these two types of emulsifiers while the entire amount of these two types of emulsifiers is fixed, then determining an appropriate proportion of the emulsifiers in terms of the stability based on the amount of creaming, turbidity, and water separation of the bottom layer after the mixture is left for one day, and then calculating the HLB value of the component (B) by using the Equation (1) below.

$$y = (x \times \text{used amount (mass\%)} + z \times \text{used amount (mass\%)})/100 \ldots \text{ Equation (1)}$$

wherein "x" represents the HLB value of the component (B), "y" represents the HLB value of the liquid paraffin, and "z" represents the HLB value of the sorbitan monostearate (NIKKOL SS-10).

Note that the HLB value of the liquid paraffin can be determined by the similar method by a combination of sorbitan monostearate (NIKKOL SS-10, HLB 4.7) and POE sorbitan monostearate (NIKKOL TS-10, HLB 14.9).

This measurement method can be also applied for polyglycerol fatty acid esters.

To clearly indicate the difference from other HLB value calculation methods, the HLB value calculated based on the method described above is referred to as HLB (NIKKOL).

In a case where the polyoxyethylene alkyl ether does not satisfy that the cloud point of 90°C or higher and the number of carbons of the hydrophobic group moiety of 16 or greater or the molecular weight of 1000 or greater and the HLB (NIKKOL) of 16 or greater, although it is possible to emulsify the component (a1) and the component (a2), polymerization does not occur because the emulsion breakage occurs before the temperature reaches the temperature required for the polymerization, or emulsion breakage occurs during the polymerization. Specific examples of the preferred nonionic surfactant include polyoxyethylene cetyl ether EO 20 (ethylene oxide 20 mol adduct), polyoxyethylene stearyl ether EO 20 (ethylene oxide 20 mol adduct), polyoxyethylene behenyl ether EO 30 (ethylene oxide 30 mol adduct), and the like.

In a case where the polyglycerol fatty acid ester does not satisfy the repeating unit of glycerin of 6 or greater and the HLB (NIKKOL) of 12 or greater, emulsification of the component (a1) and the component (a2) is difficult, and a stable emulsion cannot be obtained. Specific examples of the preferable nonionic surfactant include polyglyceryl monolaurate (polyglyceryl-6 laurate and polyglyceryl-10 laurate), polyglyceryl monostearate (polyglyceryl-10 stearate), and the like.

Specific examples of the cationic surfactant include alkyltrimethylammonium chloride, stearyltrimethylammonium chloride, lauryltrimethylammonium chloride, cetyltrimethylammonium chloride, beef tallow alkyltrimethylammonium chloride, behenyltrimethylammonium chloride, stearyltrimethylammonium bromide, behenyltrimethylammonium bromide, distearyldimethylammonium chloride, dicocoyldimethylammonium chloride, dioctyldimethylammonium chloride, di(POE)oleylmethylammonium (2 EO) chloride, benzalkonium chloride, alkyl benzalkonium chloride, alkyl dimethylbenzalkonium chloride, benzethonium chloride, stearyl dimethylbenzylammonium chloride, lanolin derivative quaternary ammonium salt, diethylaminoethylamide stearate, dimethylaminopropylamide stearate, behenic acid amide propyldimethyl hydroxypropylammonium chloride, stearoyl colaminoformyl methylpyridinium chloride, cetylpyridinium chloride, tall oil alkylbenzyl hydroxyethylimidazolinium chloride, and benzylammonium salt.

Specific examples of the amphoteric surfactant include imidazoline-type, amidobetaine-type, alkylbetaine-type, alkylamidobetaine-type, alkylsulfobetaine-type, carbobetaine-type, phosphobetaine-type, aminocarboxylic acid-type, and amidoamino acid-type amphoteric surfactants, such as N-acylamidepropyl-N,N-dimethylammoniobetaines, N-acylamidepropyl-N,N'-dimethyl -N'-β-hydroxypropylammoniobetaines, and the like. Specific examples thereof include imidazoline-type amphoteric surfactants such as sodium 2-undecyl-N,N,N-(hydroxyethylcarboxymethyl)-2-imidazoline and disodium 2-cocoyl-2-imidazolinium hydroxide-1-carboxyethyloxy salt; alkylbetaine-type amphoteric surfactants such as lauryl dimethylaminoacetic betaine and myristyl betaine; amidobetaine-type amphoteric surfactants such as coconut oil fatty acid amidopropyl dimethylamino acetic acid betaine, palm kernel oil fatty acid amidopropyl dimethylamino acetic acid betaine, beef tallow fatty acid amidopropyl dimethylamino acetic acid betaine, hardened beef tallow fatty acid amidopropyl dimethylamino acetic acid betaine, lauric acid amidopropyl dimethylamino acetic acid betaine, myristic acid amidopropyl dimethylamino acetic acid betaine, palmitic acid amidopropyl dimethylamino acetic acid betaine, stearic acid amidopropyl dimethylamino acetic acid betaine, and oleic acid amidopropyl dimethylamino acetic acid betaine; phosphobetaine-type amphoteric surfactants such as hydroxy phosphobetaine; and amidoamino acid-type amphoteric surfactants such as sodium N-lauroyl-N'-hydroxyethyl-N'-carboxymethyl ethylenediamine, sodium N-oleoyl-N'-hydroxyethyl-N'-carboxymethyl ethylenediamine, sodium N-cocoyl-N'-hydroxyethyl-N'-carboxymethyl ethylenediamine, potassium N-lauroyl-N'-hydroxyethyl-N'-carboxymethyl ethylenediamine, potassium N-oleoyl-N'-hydroxyethyl-N'-carboxymethyl ethylenediamine, sodium N-lauroyl-N-hydroxyethyl-N'-carboxymethyl ethylenediamine, sodium N-oleoyl-N-hydroxyethyl-N'-carboxymethyl ethylenediamine, sodium N-cocoyl-N-hydroxyethyl-N'-carboxymethyl ethylenediamine, monosodium N-lauroyl-N-hydroxyethyl-N',N'-dicarboxymethyl ethylenediamine, monosodium N-oleoyl-N-hydroxyethyl-N',N'-dicarboxymethyl ethylenediamine, monosodium N-cocoyl-N-hydroxyethyl-N',N'-dicarboxymethyl ethylenediamine, disodium N-lauroyl-N-hydroxyethyl-N',N'-dicarboxymethyl ethylenediamine, disodium N-oleoyl-N-hydroxyethyl-N',N'-dicarboxymethyl ethylenediamine, disodium N-cocoyl-N-hydroxyethyl-N',N'-dicarboxymethyl ethylenediamine, and the like. Furthermore, the weight average molecular weight is preferably 100 or greater.

Examples of semipolar surfactant include alkylamine oxide-type surfactants, alkylamine oxides, alkylamide amine oxides, alkylhydroxyamine oxides, and the like. Alkyldimethylamine oxides having from 10 to 18 carbons, alkoxyethyl dihydroxyethylamine oxides having from 8 to 18 carbons, and the like are preferably used.

As the reactive anionic surfactant, a reactive surfactant, which has a radically polymerizable group, in which the hydrophilic group is a carboxylate, and in which the counterion is Na+, K+, or NH4+ and which has no sulfuric acid from the perspective of skin irritation, is preferable. Furthermore, the type of the radically polymerizable group thereof may be a straight chain type or a branched structure, such as an alkenyl group and a (meth)acryl group, and a reactive anionic surfactant having these is preferable. Furthermore, besides the hydrophilic group, a nonionic hydrophilic group such as ethylene oxide may be bonded.

When the reactive nonionic surfactant is used, the reactive nonionic surfactant includes a radically polymerizable group such as an alkenyl group and a (meth)acryl group having a straight chain or a branched structure, the hydrophilic group moiety is formed from ethylene oxide or propylene oxide or butylene oxide. The hydrophilic group may be only ethylene oxide, or may be a combination of ethylene oxide and propylene oxide or butylene oxide. The cloud point thereof is preferably 70°C or higher, more preferably 80°C or higher, and even more preferably 100°C or higher. In a case where the cloud point is lower than 70°C, emulsification capacity of the nonionic surfactant is impaired, problems occur in stability of the emulsion obtained by emulsifying a monomer during the radical polymerization, and a uniform emulsion may not be obtained.

The amount of the component (B) surfactant used is preferably from 0.1 to 30 parts by weight, and more preferably from 0.1 to 10 parts by weight, per 100 parts by weight of the component (A). In a case where the amount exceeds 30 parts by weight, performance of the component (A) may become insufficient. Furthermore, in a case where the amount is less than 0.1 parts by weight, the component (A) cannot be stably emulsified.

In a case where an amphoteric surfactant, a semipolar surfactant, a reactive surfactant, and a high molecular weight emulsifier are also included, the compounded amount thereof is preferably from 0.1 to 30 parts by weight, and more preferably from 0.1 to 10 parts by weight, per 100 parts by weight of the component (A). In a case where the amount exceeds 30 parts by weight, performance of the component (A) may become insufficient. Furthermore, in a case where the amount is less than 0.1 parts by weight, the component (A) cannot be stably emulsified.

The weight average molecular weight of the high molecular weight emulsifier to be used is preferably from

40000 to 3000000, and more preferably from 300000 to 2750000, and even more preferably from 2000000 to 2500000. Specific examples of the anionic polymer as an anionic polymer having a saccharide backbone include commercially available products such as Na stearoxy PG-hydroxyethylcellulose sulfonate (INCI: SODIUM STEAROXY PG-HYDROXYETHYLCELLULOSE SULFONATE) (POIZ 310 (Kao Corporation)) as well as xanthan gum, carrageenan, locust bean gum, and the like. Furthermore, examples of the polymer of acrylic acid and/or methacrylic acid include alkyl acrylate-alkyl methacrylate-polyoxyethylene (20) stearyl ether copolymers (INCI: ACRYLATES/STEARETH-20 METHACRYLATE COPOLYMER), alkyl acrylate-alkyl methacrylate-polyoxyethylene (25) lauryl ether copolymers (INCI: ACRYLATES/LAURETH-25 METHACRYLATE COPOLYMER), alkyl acrylate-alkyl methacrylate-polyoxyethylene (25) behenyl ether copolymers (INCI: ACRYLATES/BEHENETH-25 METHACRYLATE COPOLYMER), acrylic acid-alkyl methacrylate copolymers (INCI: ACRYLATES/C10-30 ALKYL ACRYLATE CROSSPOLYMER), acrylic acid-vinyl neodecanoate copolymers (INCI: ACRYLATES/VINYL NEODECANOATE CROSSPOLYMER), (alkyl acrylate-octylacrylamide) copolymers (INCI: ACRYLATES/OCTYLACRYLAMIDE COPOLYMER), (acrylate-steareth-20 itaconate) copolymers (INCI: ACRYLATES/STEARETH-20 ITACONATE COPOLYMER), (acrylate/ceteth-20 itaconate) copolymers (INCI: ACRYLATES/CETETH-20 ITACONATE COPOLYMER), (acrylate/amino acrylate/C10-30 alkyl PEG-20 itaconate) copolymers (INCI: ACRYLATES/AMINOACRYLATES/C10-30ALKYL PEG-20 ITACONATE COPOLYMER), and the like.

As these polymers, commercially available products such as ACULYN 88, ACULYN 22, ACULYN 28, and ACULYN 38 (Rohm and Haas Japan), Carbopol ETD 2020, Carbopol Ultrez 21, Carbopol Ultrez 20, PEMULEN TR-1, and PEMULEN TR-2 (Lubrizol Advanced Materials), STRUCTURE 2001, STRUCTURE 3001, STRUCTURE PLUS, and DERMACRYL 79 (Nippon NSC Ltd.), and the like can be used.

Examples of the nonionic polymer include hydroxyethyl cellulose, hydroxypropyl methylcellulose, hydroxypropyl guar gum, polyvinylpyrrolidone, polyethylene glycol, and the like. The weight average molecular weight of these is preferably from 40000 to 3000000, and more preferably from 300000 to 2750000, and even more preferably from 2000000 to 2500000.

As these nonionic polymers, commercially available products, such as ALKOX series (Meisei Chemical Works, Ltd., polyethylene glycol): ALKOX E-30 (weight average molecular weight: 300000 to 500000), ALKOX E-45 (weight average molecular weight: 600000 to 800000), ALKOX E-60 (weight average molecular weight: 1000000 to 1200000), ALKOX E-75 (weight average molecular weight: 2000000 to 2500000), and ALKOX E-100 (weight average molecular weight: 2500000 to 3000000); Metolose series (Shin-Etsu Chemical Co., Ltd., hydroxypropyl methylcellulose): Metolose 60SH-10000 (weight average molecular weight: 380000); JAGUAR HP series (Rhodia, hydroxypropyl guar gum): JAGUAR HP8, HP105 and HP-120 (each weight average molecular weight of these: 2200000), and the like can be used.

Examples of the amphoteric polymer include a copolymer of dimethyl diallyl ammonium chloride, acrylic acid, and acrylamide (trade name: Merquat PLUS 3330 and Merquat PLUS 3331; available from ONDEO Nalco), Merquat S (weight average molecular weight: 260000; cationic charge density: 4.22 meq/g; DMDAAC:AM = 50:50), and the like.

The weight average molecular weight of the component (B) is preferably from 250 to 6000, and more preferably from 500 to 3000, from the perspectives of excellent waterproofness of the film and excellent stability of the emulsion particles.

During the process of producing a cosmetic composition of an embodiment of the present invention, by subjecting the component (A) to emulsification by using the component (B), a cosmetic composition in a form of emulsion containing at least the components (A) and (B) is obtained. The volume average particle size of the component (A) after the emulsion of this cosmetic composition (measured by the laser diffraction/scattering method or the like) is preferably 500 nm or less, and more preferably 300 nm or less. Furthermore, after the component (A) is emulsified by the component (B), the amount of the component (A) in the oil phase of the emulsion particle is 50 wt.% or greater but preferably 70 wt.% or greater. The obtained cosmetic composition can be used as a cosmetic raw material composition, a composition for an oil-in-water cosmetic, and a composition for a water-in-oil cosmetic. Furthermore, the obtained cosmetic composition can be used as a raw material composition for a cosmetic together with other components; however, this cosmetic composition exhibits characteristics as the cosmetic composition of an embodiment of the present invention especially in an oil-in-water cosmetic.

The component (X) water is not particularly limited as long as the water is water typically used in cosmetic materials, quasi drugs, and pharmaceutical preparations. For example, purified water such as distilled water and ion exchanged water, saline, a phosphoric acid buffer aqueous solution, and the like can be used. Furthermore, the content of the component (X) in the cosmetic composition is at least 50 parts, 70 parts, or 100 parts, and more preferably 200 parts, per 100 parts by weight of the component (A) from the perspective of emulsion formation. Furthermore, the upper limit thereof is 2000 parts, 1500 parts, or 1200 parts, preferably 1000 parts, and more preferably 900 parts, for the same reason.

The composition of an embodiment of the present invention containing the film forming agent is preferably used as a cosmetic composition, and is preferably used for a cosmetic using the same. Furthermore, among cosmetics, the composition can be used for a skin cosmetic and/or a hair cosmetic.

The component (C) cosmetically acceptable medium used in an embodiment of the present invention contains the components from (c1) to (c4) or, without limitation to these, the component (C) may contain at least one component selected from the group consisting of volatile or non-volatile carbon-based, hydrocarbon-based, fluoro and/or silicone oils and/or solvents of mineral, animal, plant, or synthetic origin; fatty substances that are solid at room temperature, especially waxes, paste-like fatty substances, and gums; water; hydrophilic organic solvents; dyes; polymers; vitamins, thickeners, gelling agents, trace elements, softeners, sequestering agents, fragrances, acidifying or basifying agents, antiseptics, sunscreens, surfactants, antioxidants, hair-loss counteractants, antidandruff agents, propellants, ceramides, film forming auxiliary agents, and mixtures thereof.

In a case where an anionic surfactant is used as the surfactant, the cosmetic composition of an embodiment of the present invention works well with a salt with a low valency from the perspective of stability of emulsion. Furthermore, in a case where a salt with a valency of 2 or greater is used, it is preferable to mix a phase containing the salt is mixed to emulsify an oil phase and an aqueous phase and then mix the composition of an embodiment of the present invention.

(c1): UV absorbent

The component (c1) used in an embodiment of the present invention includes an oil-soluble UV absorbent and a water-soluble UV absorbent. Examples of the oil-soluble UV absorbent include 2-ethylhexyl paramethoxycinnamate (Uvinul MC80; manufactured by BASF), 2,4,6-tris[4-(2-ethylhexyloxycarbonyl)anilino]-1,3,5-triazine (Uvinul T150; available from BASF), 4-tert-butyl-4'-methoxydibenzoylmethane (PARSOL 1789; available from DSM Nutrition Japan KK), octocrylene (PARSOL 340; available from DSM Nutrition Japan KK), 2-ethylhexyl dimethoxybenzylidene dioxoimidazolidine propionate (Soft Shade DH; available from Ajinomoto), diethylamino hydroxybenzoyl hexyl benzoate (Uvinul A Plus; available from BASF), bisethylhexyloxyphenol methoxyphenyl triazine (Tinosorb S; available from BASF), methylene bis-benzotriazolyl tetramethylbutylphenol (Tinosorb M; available from BASF).

Furthermore, examples of the water-soluble UV absorbent include phenylbenzimidazole sulfonic acid, 3,4,5-trimethoxycinnamic acid N-2-(2-hydroxyethoxy)ethyl amide, 4-methoxycinnamic acid N-2-(2-hydroxyethoxy)ethylamide, and 2-hydroxy-4-methoxy-5 -sulfoxoniumbenzophenone sodium.

From the perspective of UV absorbing effect, one type or a combination of two or more types of these UV absorbents is preferable. Furthermore, the content of the UV absorbent is in a range from 0.1 to 30 wt.%, preferably in a range from 0.5 to 25 wt.%, and more preferably in a range from 1 to 20 wt.%, relative to the amount of the entire composition of the cosmetic.

(c2): Inorganic powder

The component (c2) inorganic powder used in an embodiment of the present invention include a pigment and a UV protection agent. Examples of the pigment include a coloring pigment and an extender. Examples of the coloring pigment include a pigment-grade titanium oxide, zinc oxide, aluminum oxide, barium sulfate, calcium carbonate, iron oxide, and carbon black. Examples of the extender include talc, mica, sericite, and the like. Although the particle size thereof is not particularly limited but is preferably a particle size that is smaller than the particle size of the emulsion particle in the cosmetic.

Furthermore, the UV protection agent has a particle size of less than 0.3 μm, and includes a hydrophobization-treated zinc oxide and/or a hydrophobization-treated titanium oxide. The particle size herein is a volume-based average particle size measured by using a laser diffraction particle size distribution measurement device and using an organic solvent such as ethanol as a solvent.

These are used to add a coloration to a cosmetic material or to reflect UV radiation.

One or more types may be used as the component (c2), and the amount of the component (c2) is from 0.1 to 25 wt.%, and preferably from 2.0 to 10 wt.%, relative to the amount of the entire composition of the cosmetic. In a case where the amount is less than 0.1 wt.%, smooth finish cannot be obtained, and in a case where the amount is greater than 30 wt.%, texture during usage becomes poor.

In addition to those described above, the cosmetic of an embodiment of the present invention may contain powder that is used in typical cosmetics, without particular limitation of shape. Powder of a shape such as spherical, plate-like, or needle-like shape; particle size such as aerosol-like, microparticle, or pigment-grade particle size; particle structure such as porous or non-porous; and the like may be used.

Examples of the powder having hydrophobicity by itself include nylon powder, polymethyl methacrylate, powder of acrylonitrile-methacrylic acid copolymer, powder of vinylidene chloride-methacrylic acid copolymer, polyethylene powder, polystyrene powder, organopolysiloxane elastomer powder, polymethylsilsesquioxane powder, polytetrafluoroethylene powder, urethane powder, wool powder, magnesium stearate, zinc stearate, N-acyl lysine, organo-modified clay materials, boron nitride, organic tar pigments, and the like.

Examples of the powder that is not hydrophobic by itself include titanium oxide, Prussian blue, ultramarine, red

iron oxide, yellow iron oxide, black iron oxide, zinc oxide, aluminum oxide, silicon dioxide, magnesium oxide, zirconium oxide, magnesium carbonate, calcium carbonate, chromium oxide, chromium hydroxide, aluminum silicate, magnesium silicate, aluminum magnesium silicate, mica, synthetic mica, synthetic sericite, sericite, talc, kaolin, barium sulfate, bentonite, smectite, and bismuth oxychloride; and composite powders such as titanated mica, iron oxide-coated mica, iron oxide-coated titanated mica, fine particulate titanium oxide-coated titanated mica, fine particulate zinc oxide-coated titanated mica, red iron oxide-coated titanated mica, barium sulfate-coated titanated mica, titanium oxide-containing glass flakes, zinc oxide-containing silicon dioxide, and the like.

When subjecting a powder that is not hydrophobic by itself to hydrophobization treatment, a publicly known hydrophobizing agent and a publicly known hydrophobizing method may be used. For example, dry treatment, wet treatment, or the like may be performed by using a surface treatment agent, such as a fluorine compound, a silicone-based compound, a metal soap, and an oil. In addition, surface hydrophobizing method by using a silane-based treating agent is also possible. Specific examples of the surface treating agent include fluorine-based compounds such as perfluoroalkylphosphates and perfluoroalkylalkoxysilanes, silicone-based compounds such as methylhydrogenpolysiloxane, cyclic silicones, and organopolysiloxanes modified with a trialkoxy group at one or both ends thereof, metal soaps such as aluminum stearate and zinc stearate, and amino acid-based compounds such as lauroyl lysine. Furthermore, additionally, the silane-based treating agent to be used may be a silane-based treating agent that hydrophobizes the surface by utilizing a reaction with the inorganic powder, and preferably a silane-based treating agent that can impart a trimethylsilyl group, a dimethylsilyl group, or a monomethylsilyl group to the surface of the inorganic powder. Examples of the silane-based treating agent that can impart a trimethylsilyl group include trimethylchlorosilane and hexamethyldisilane. Examples of the silane-based treating agent that can impart a dimethylsilyl group include dimethyldichlorosilane, dimethyldimethoxysilane, and polydimethylsilazane. Examples of the silane-based treating agent that can impart a monomethylsilyl group include methyltrichlorosilane, methyltrimethoxysilane, octylsilane, and perfluoroalkylsilane. In addition, a silane coupling agent having an organic functional group, such as γ-(2-aminoethyl)aminopropyltrimethoxysilane, γ-methacryloxypropyltrimethoxysilane, γ-glycidoxypropyltrimethoxysilane, vinyltriacetoxysilane, γ-mercaptopropylmethyldimethoxysilane, can be used. In addition to the silane-based treatment, other examples of treatment include organosiloxane treatments such as a methylhydrogenpolysiloxane treatment, a silicone resin treatment, a silicone gum treatment, an acryl silicone treatment, and a fluorinated silicone treatment; metal soap treatments such as a zinc stearate treatment; fluorine compound treatments such as a perfluoroalkyl phosphate treatment and a perfluoro polyether treatment; amino acid treatments such as an N-lauroyl-L-lysine treatment; oil agent treatments such as a squalane treatment; acryl treatments such as an alkyl acrylate treatment; and the like, and a combination of one or more types can be used. Note that the amount of the surface treatment is not limited, the amount is preferably from 0.5 to 20 wt.%, and more preferably from 2 to 8 wt.%, relative to the amount of the original powder.

(c3): Hydrophilic thickener

The stability of the cosmetic of an embodiment of the present invention is further enhanced by further blending a component (c3) hydrophilic thickener in addition to the components (A) and (B) that are essential in the composition described above. The hydrophilic thickener used in an embodiment of the present invention is not particularly limited as long as the hydrophilic thickener is used in cosmetic materials, and examples thereof include natural water-soluble polymers, semi-synthetic water-soluble polymers, synthetic water-soluble polymers, inorganic water-soluble polymers, and the like.

Specific examples of the natural water-soluble polymer include plant polymers, such as gum arabic, tragacanth gum, galactan, guar gum, carob gum, karaya gum, carrageenan, pectin, agar, quince seed (Cydonia oblonga), algae colloid (brown algae extract), starch (rice, corn, potato, wheat), and glycyrrhizic acid; microbial polymers, such as xanthan gum, dextran, succinoglucane, and pullulan; animal polymers, such as collagen, casein, albumin, and gelatin; and the like.

Examples of the semi-synthetic water-soluble polymer include starch-based polymers such as carboxymethyl starch and methylhydroxypropyl starch; cellulose-based polymers such as methyl cellulose, nitrocellulose, ethyl cellulose, methylhydroxypropyl cellulose, hydroxyethyl cellulose, sodium cellulose sulfate, hydroxypropyl cellulose, sodium carboxymethyl cellulose (CMC), crystalline cellulose, and cellulose powder; alginic acid-based polymers, such as sodium alginate and propyleneglycol alginate, and the like.

Examples of the synthetic water-soluble polymer include vinyl polymers such as polyvinyl alcohol, polyvinyl methyl ether, polyvinyl pyrrolidone, and carboxyvinyl polymers (carbomers); polyoxyethylene-based polymers such as polyethylene glycol (molecular weight: 1500, 4000, 6000); polyoxyethylene-polyoxypropylene copolymers; acrylic polymers such as sodium polyacrylate, polyethyl acrylate, polyacrylamide, and acrylic acid-methacrylic acid alkyl copolymers (trade name: PEMULEN TR-1); polyethyleneimine; cationic polymers; and the like. Examples of the inorganic water-soluble polymer include bentonite, AlMg silicate (trade name: VEEGUM), laponite, hectorite, silicic anhydride, and the like.

In particular, when a skin-lightening agent of a salt-type that easily becomes unstable over time, such as 4-methoxysalicylate, is formulated, the stability can be significantly enhanced by blending one type or two or more types

of hydrophilic thickeners selected from the group consisting of xanthan gum, crosslinked N,N-dimethylacrylamide-2-acrylamide-2-methylpropanesulfonic acid sodium salt copolymers, and carboxyvinyl polymers.

The acrylic acid-based polymer needs to be a homopolymer having a constituent unit selected from the following monomers or a copolymer containing two or more types of the following monomers: methacrylic acid, acrylic acid, methacrylate, acrylate, methacrylamide, and acrylamide. Examples of the acrylic acid-based polymer include carboxyvinyl polymers (Shintaren K, L; available from Wako Pure Chemical Industries, Ltd.), alkyl-modified carboxyvinyl polymers (PEMULEN TR-1, TR-2; available from The Lubrizol Corporation), polyacrylamide sold by SEPPIC (SEPIGEL 305), (Na acrylate/Na acryloyldimethyltaurate) copolymers (SIMULGEL EG), (hydroxyethyl acrylate/Na acryloyldimethyltaurate) copolymers (SIMULGEL FL, SIMULGEL NS, SEPIPLUS S, SEPINOV EMT 10), (acrylamide/ammonium acrylate) copolymers (SEPIPLUS 265), polyacrylate-13 (SEPIPLUS 400), and the like. One type or two or more types of the acrylic acid-based polymers selected from these can be used. Among these, the case where one type or two or more types of the alkyl-modified carboxyvinyl polymer and (Na acrylate/Na acryloyldimethyltaurate) copolymers is used is preferable from the perspectives of stable formulation even when a small amount is used, prevention effect of unevenness during application, and enhancement in texture during usage.

The compounded amount of the hydrophilic thickener in the cosmetic of an embodiment of the present invention is preferably from 0.1 to 5 wt.%, more preferably from 0.3 to 1.5 wt.%, and even more preferably from 0.5 to 1 wt.%, in the amount of the entire composition of the cosmetic. When the compounded amount is less than 0.1 wt.%, the stabilizing effect due to the addition may not be achieved, and when the compounded amount is greater than 5 wt.%, stickiness or heavy feel during the application may be caused.

(c4): Polyether-modified silicone

The polyether-modified silicone of the optional component (c4) that can be used in an embodiment of the present invention is a component that may be partially the same as the nonionic surfactant described above depending on the function in the cosmetic or the cosmetic composition and is a substance in which a polyoxyalkylene group, preferably a polyoxyethylene group or a poly(oxyethylene-oxypropylene), is bonded to a dimethylpolysiloxane. Polyether-modified silicones having various bonding positions, HLBs, and viscosities have been known. As the bonding positions, side chain, one end, and both ends of silicone chains have been known. Also, a substance in which a silicone chain and a polyoxyalkylene chain are bonded to a main chain in blocks has been known. In an embodiment of the present invention, a polyether-modified silicone with any type of bonding positions is used; however, a side chain-type or block-type bonding position is preferable.

The HLB value of the polyether-modified silicone is specified by the Griffin method, and is from 1 to 6, and particularly preferably from 2 to 5. The viscosity at 25°C is preferably from 20 to 100000 mm2/s, and more preferably from 50 to 50000 mm2/s. Note that, as the viscosity, the viscosity value (mPa·s) measured for 1 minute by using a B-type viscometer is taken as the kinematic viscosity (mm2/s) as it stands, wherein a No. 3 rotor is used at 12 rpm (1000 mm2/s or greater but less than 10000 mm2/s).

Specific examples of the component (c4) polyether-modified silicone include silicone KF-6015 (HLB 4.5, viscosity: 150 mm2/s), KF-6017 (HLB 4.5, viscosity: 600 mm2/s, available from Shin-Etsu Chemical Co., Ltd.), KF-6019 (HLB 4.5, viscosity: 850 mm2/s, available from Shin-Etsu Chemical Co., Ltd.), KF-6028 (HLB 4, viscosity: 900 mm2/s, available from Shin-Etsu Chemical Co., Ltd.), SH-3772M (HLB 6, viscosity: 1050 mm2/s, available from Dow Corning Toray Co., Ltd.), SH-3775M (HLB 5, viscosity: 1600 mm2/s, available from Dow Corning Toray Co., Ltd.), FZ-2233 (HLB 2, viscosity: 5000 mm2/s), and the like.

One or more types of these polyether-modified silicone can be used, and the amount of the polyether-modified silicone is from 0.1 to 6 wt.%, and preferably from 0.3 to 2.4 wt.%, relative to the amount of the entire composition. When the amount is less than 0.1 wt.%, sufficient ease in fixing makeup cannot be achieved, and when the amount is greater than 6 wt.%, the long-lasting characteristics tend to be deteriorated.

The weight ratio of the component (A) to the component (c4) is preferably (A)/(c4) = from 1.2 to 30, more preferably from 1.4 to 25, and even more preferably from 2.3 to 12.5. When the weight ratio is less than 1.2, it is difficult to fix makeup, and when the amount is greater than 30, unevenness in coating occurs, and smooth finish cannot be obtained.

Oil

Furthermore, examples of the oil contained in the present cosmetic include polar oils, non-polar oils, volatile oils, and the like. For example, the polar oil is a polar oil that is liquid at 25°C. Note that the liquid indicates a condition having a fluidity and includes cream and paste.

The polar oil is not particularly limited as long as the polar oil is used in typical cosmetics but is preferably a polar oil having the solubility parameter (SP value) of 19 or greater, and particularly preferably from 19 to 23. The non-polar oil is preferably a non-polar oil having the solubility parameter (SP value) of less than 19, and particularly preferably from 12 to 16.

Note that the SP value of the polar oil is the solubility parameter δ and is a material constant given by δ = (E/V)1/2 (J/cm3) wherein E is the molecular cohesive energy and V is the molecular volume of the liquid. Although the SP value can be determined by various methods, in the present invention, the SP value is determined in accordance with the Fedors method (Brandrup, J. Polymer Handbook 4th; John Wiley & Sons, Inc., 1999), with the parameters described in pp. VII 685 to 686.

Specific examples thereof include isotridecyl isononanoate (16.5), diisostearyl malate (17.9), isostearyl myristate (16.9), triisostearin (17.1), tris-ethoxydiglycol phosphate (18.1), neopentyl glycol dicaprate (18.2), glyceryl tri(2-ethylhexanoate) (18.6), diglyceryl diisostearate (18.7), glyceryl monoisostearate monomyristate (19.1), 2-ethylhexyl paramethoxycinnamate (19.2), dl-α-tocopherol (19.4), methylphenylpolysiloxane (20.0), acetyltributyl citrate (20.1), polyglyceryl-2 isostearate (21.7), and the like.

One or more types of these oils may be used and the amount of the oil is from 0.1 to 30 wt.%, and preferably from 0.5 to 10 wt.%, relative to the amount of the entire composition in the cosmetic. When the amount is less than 0.1 wt.%, sufficient effect cannot be achieved, and when the amount is greater than 30 wt.%, it is difficult to fix makeup.

Hydrophilic microparticle

The hydrophilic microparticle optionally used in the cosmetic of an embodiment of the present invention is treated with one or a plurality of materials, and may be surface-coated with a hydrophilic substance. Examples of the hydrophilic treatment material include hydrophilic polymers, cellulose, agar, deoxyribonucleic acid, lecithin, surfactants, polyacrylic acid, inorganic substances such as silica, alumina, and zirconia, and preferably, the hydrophilic treatment material is used to impart hydrophilicity to a microparticle after a plurality of processes or treatments. The hydrophilic microparticle may be in any form (shapes, such as spherical, rod-like, needle-like, plate-like, irregular, flake-like, and spindle-like) and in any state (smog, microparticle, pigment, and the like), and may have any particle structure (porous or non-porous). The hydrophilic microparticle may be inorganic powder or organic powder, a surfactant metal salt microparticle, an organic pigment, a pearl pigment, or a metal powder pigment. Examples of the hydrophilic microparticle include silica hydrate, zinc oxide, titanium dioxide, cerium oxide, zirconium dioxide, aluminum hydroxide, sodium metaphosphate, and cellulose. Zinc oxide or titanium dioxide or cerium oxide is preferably used. Such a hydrophilic microparticle has sun blocking effect or reflection effect and can protect skin from UV. The content of the hydrophilic microparticle in the emulsion is from 0.10 to 70 wt.%, preferably from 0.20 to 50 wt.%, and even more preferably from 0.30 to 30 wt.%.

Examples of the hydrophilic microparticle include Cosmeserve WP-40W (Dainihonkasei Co., Ltd.), FINEX-50W (Sakai Chemical Industry Co., Ltd), FINEX-30W (Sakai Chemical Industry Co., Ltd), ST-455WS (Titan Kogyo, Ltd.), ST-495M (Titan Kogyo, Ltd.), TTO-S-1 (Ishihara Sangyo Kaisha, Ltd.), TTO-S-3 (Ishihara Sangyo Kaisha, Ltd.), TTO-V-3 (Ishihara Sangyo Kaisha, Ltd.), MT-150W (Tayca Corporation), MT-100AQ (Tayca Corporation), and MT-100WP (Tayca Corporation).

In addition to the components described above, the cosmetic of an embodiment of the present invention may contain components used in typical cosmetics in qualitative and quantitative ranges that do not impair the object and effect of the present invention, and examples of such a component include oily substances except those described above, surfactants, pigments, water-soluble polymers, antioxidants, fragrances, pigments, antiseptics, UV absorbents, thickeners, pH adjusting agents, blood circulation promoters, cooling sensation agents, antiperspirants, germicides, skin activators, moisturizers, refreshing agents, and the like. Furthermore, the type of cosmetic and the form of formulation are not particularly limited and may be skin cosmetic materials such as skin care products, antiperspirant products, deodorant products, makeup products, and UV blocking products; hair cosmetic materials such as eyelash cosmetic products, hair washing products, hair dressing products, hair coloring products, hair tonic products, hair rinse products, hair conditioner products, and hair treatment products; and hair cosmetic products such as cosmetics for bath.

The external preparations are applied for skin, nail, hair, and the like of human body and, for example, can be used for treatment of various disorders by blending a pharmaceutically active component. The cosmetics are also applied for skin, nail, hair, and the like of human body but are used for cosmetic purposes. Even when the purpose is for an "external preparation", the external preparation may be practically used in the same use and the same dose as cosmetics. Therefore, for the cosmetic of an embodiment of the present application, such an external preparation is described as being included in cosmetics. Examples thereof include antiperspirants, skin washing agents, skin external preparations, hair washing agents, hair external preparations, and the like. Examples of the use as a medicament of external preparation thereof include hair regrowth agents, hair growth promoters, analgesics, germicides, antiinflammatory agents, refreshing agents, and skin anti-aging agents, but are not limited to these.

The antiperspirant, skin washing agent, skin external preparation, or skin cosmetic according to an embodiment of the present invention contain a low-odor glycerin derivative-modified silicone of an embodiment of the present invention,

and the forms thereof are not particularly limited and may be solution, emulsion, cream, solid, semisolid, paste, gel, powder, multilayered, mousse, water-in-oil or oil-in-water emulsion compositions (emulsion compositions). Specific examples of the skin external preparation or the skin cosmetic according to an embodiment of the present invention include foundation cosmetic products such as skin lotions, milky lotions, creams, sunscreen milky lotions, sunscreen creams, hand creams, cleansing agents, massage lotions, cleansing agents, antiperspirants, and deodorants; and make-up cosmetic products, such as foundations, make-up bases, blushers, rouges, eye shadows, eye liners, mascaras, nail enamels.

Wrinkle smoothing effect

To achieve wrinkle smoothing effect, the cosmetic composition according to an embodiment of the present invention can be used for skin cosmetics and skin external preparations. The cosmetic composition is effective for cosmetic materials for wrinkle, such as eye creams and cosmetics for mouth, because the irregularities of skin may be made unnoticeable due to stretching effect of the film formed on the skin.

Furthermore, an embodiment of the present invention can be employed as a film forming agent besides cosmetic compositions and cosmetics. That is, an embodiment of the present invention can be employed without limitation, as long as it may be employed in applications where water repellency effects, oil repellency effects, integrity, strength, and rub resistance are required for a film. In particular, an embodiment of the present invention may be employed in coating compositions, water repellency-imparting agents for fibers, treatment agents for inorganic materials/organic materials. Furthermore, because the film forming agent of an embodiment of the present invention can be used in an aqueous environment, handling thereof is very easy.

[Examples]

The present invention is described in detail below by examples and comparative examples; however, these examples do not limit the present invention described in the claims. In the examples, the viscosity is the measured value at 25°C, "part" representing the compounded amount means "part by weight", "%" representing the content means "wt.%".

Example 1

Monomer emulsification

In a beaker 1, 0.53 parts by weight of sodium lauryl sulfate was weighed, and then 51.72 parts by weight of ion exchanged water was added and agitated to form an aqueous solution. In a beaker 2, 10.09 parts by weight of methyl methacrylate, 3.18 parts by weight of butyl acrylate, and 13.23 parts by weight of carbosiloxane dendrimer represented by General Formula (1) were added and homogenized. After the mixture in the beaker 2 was added to the beaker 1 and then agitated for several minutes, it was passed through a pressure of 400 to 500 kg/cm$^2$ for multiple times by using a homogenizer to obtain a milky white monomer emulsion having an average particle size of 153 nm.

Radical polymerization

In a separable flask, 8.00 parts by weight of ion exchanged water and a part of the obtained monomer emulsion were charged, and the temperature was raised to 70°C while the mixture was agitated. After the temperature reached 70°C, the rest of the obtained monomer emulsion and 13.25 parts by weight of 1% potassium persulfate aqueous solution prepared with ion exchanged water were gradually added dropwise simultaneously to allow a reaction to proceed. After the reaction, odor of methyl methacrylate or butyl acrylate was not noticed. The completed emulsion (Run 41) had an average particle size of 143 nm and a nonvolatile content of approximately 30%, and completion of the reaction was confirmed.

Example 2

The same procedure as in Example 1 was performed except for using the following composition, changing the polymerization temperature to 80°C, changing the emulsifier from the sodium lauryl sulfate to a laureth-1 phosphate, and using the emulsifier for neutralization with 50% sodium hydroxide.

Monomer emulsification

Beaker 1:

Laureth-1 phosphate (90%): 0.67 parts by weight
50% Sodium hydroxide: 0.17 parts by weight
Ion exchanged water: 46.87 parts by weight

Beaker 2:

Methyl methacrylate: 11.49 parts by weight
Butyl acrylate: 3.61 parts by weight
Carbosiloxane dendrimer represented by General Formula (1): 15.06 parts by weight
2-Phenoxyethanol: 0.30 parts by weight
Monomer emulsion average particle size:
Milky white, and average particle size of 117 nm.

Radical polymerization

Separable flask:

Ion exchanged water: 6.75 parts by weight
Monomer emulsion
1% Potassium persulfate aqueous solution prepared with ion exchanged water: 15.08 parts by weight

Results:

No odor of methyl methacrylate or butyl acrylate was noticed.

The completed emulsion (Run 52) had an average particle size of 114 nm and a nonvolatile content of approximately 30%. The component (A) polymer had a weight average molecular weight of 1250000 measured by gel permeation chromatography by using tetrahydrofuran as a solvent and based on calibration with polystyrene.

Example 3

The same procedure as in Example 1 was performed except for using the following composition, changing the polymerization temperature to 80°C, changing the emulsifier from the sodium lauryl sulfate to a laureth-1 phosphate, and using the emulsifier for neutralization with 50% sodium hydroxide.

Monomer emulsification

Beaker 1:

Sodium lauryl phosphate: 0.60 parts by weight
Ion exchanged water: 49.72 parts by weight

Beaker 2:

Methyl methacrylate: 11.45 parts by weight
Butyl acrylate: 3.62 parts by weight
Carbosiloxane dendrimer represented by General Formula (1): 15.07 parts by weight
2-Phenoxyethanol: 0.30 parts by weight
Monomer emulsion average particle size:
Milky white, and average particle size of 125 nm.

Radical polymerization

Separable flask:

Ion exchanged water: 4.17 parts by weight
Monomer emulsion
1% Potassium persulfate aqueous solution prepared with ion exchanged water: 15.07 parts by weight

Results:

No odor of methyl methacrylate or butyl acrylate was noticed.

The completed emulsion (Run 53) had an average particle size of 109 nm and a nonvolatile content of approximately 30%.

Example 4

The same procedure as in Example 2 was performed except for using the following composition.

Monomer emulsification

Beaker 1:

Laureth-1 phosphate (90%): 0.68 parts by weight
50% Sodium hydroxide: 0.17 parts by weight
Ion exchanged water: 47.48 parts by weight

Beaker 2:

Methyl methacrylate: 18.63 parts by weight
Butyl acrylate: 5.86 parts by weight
Carbosiloxane dendrimer represented by General Formula (a1): 6.10 parts by weight 2-Phenoxyethanol: 0.31 parts by weight
Monomer emulsion average particle size:
Milky white, and average particle size of 82 nm.

Radical polymerization

Separable flask:

Ion exchanged water: 5.47 parts by weight
Monomer emulsion
1% Potassium persulfate aqueous solution prepared with ion exchanged water: 15.29 parts by weight

Results:

No odor of methyl methacrylate or butyl acrylate was noticed.

The completed emulsion (Run 55) had an average particle size of 105 nm and a nonvolatile content of approximately 30%.

Example 5

Monomer emulsification

In a beaker 1, 1.91 parts by weight of oxirane polyaddition product of {reaction product of alkanol (C = 10 to 14, branched) and 1-(allyloxy)-2,3-epoxypropane} containing 65% aqueous solution of {$\alpha$-[2-(allyloxy)-1-({[alkyl (C = 10 to 14)]oxy}methyl]ethyl)-co-hydroxypoly(n = 1 to 100)(oxyethylene)} as a main component, and 0.46 parts by weight of 30% aqueous solution of alkenyl potassium succinate were weighed. Then, 37.34 parts by weight of ion exchanged water was added to the mixture and agitated to form an aqueous solution. In a beaker 2, 10.99 parts by weight of methyl methacrylate, 3.45 parts by weight of butyl acrylate, and 14.36 parts by weight of carbosiloxane dendrimer represented by General Formula (1) were added. After the mixture in the beaker 2 was added to the beaker 1 and then agitated for several minutes, it was passed through a pressure of 400 $kg/cm^2$ for multiple times by using a homogenizer to obtain a milky white monomer emulsion having an average particle size of 170 nm.

Radical polymerization

In a separable flask, 17.11 parts by weight of ion exchanged water and a part of the obtained monomer emulsion were charged, and the temperature was raised to 60 to 80°C while the mixture was agitated. After the temperature reached 70°C, the rest of monomer emulsion and 14.25 parts by weight of 1% potassium persulfate aqueous solution prepared with ion exchanged water were gradually added dropwise simultaneously to allow a reaction. After the reaction, odor of methyl methacrylate or butyl acrylate was not noticed. The completed emulsion (Run 36) had an average particle size of 174 nm and a nonvolatile content of approximately 30%, and completion of the reaction was confirmed. The component (A) polymer had a weight average molecular weight of 1500000 measured by gel permeation chromatography by using tetrahydrofuran as a solvent and based on calibration with polystyrene.

Example 6

The same procedure as in Example 1 was performed except for using the following composition, changing the polymerization temperature to 65°C, and changing the emulsifier from the sodium lauryl sulfate to an ethylene oxide 20 mol adduct of polyoxyethylene (C16) ether. The HLB (NIKKOL) of the ethylene oxide 20 mol adduct of polyoxyethylene (C16) ether in Example 6 was 17.0, the molecular weight was 1122, and the cloud point was 100°C or higher.

Monomer emulsification

Beaker 1:

Ethylene oxide 20 mol adduct of polyoxyethylene (C16) ether: 2.90 parts by weight
Ion exchanged water: 52.55 parts by weight

Beaker 2:

Methyl methacrylate: 9.57 parts by weight
Butyl acrylate: 4.50 parts by weight
Carbosiloxane dendrimer represented by General Formula (a1): 14.50 parts by weight
2-Phenoxyethanol: 0.58 parts by weight
Monomer emulsion average particle size:
Milky white, and average particle size of 121 nm.

Radical polymerization

Separable flask:

Ion exchanged water: 0.47 parts by weight
Monomer emulsion

1.5% Potassium persulfate aqueous solution prepared with ion exchanged water: 14.50 parts by weight

Results:

No odor of methyl methacrylate or butyl acrylate was noticed.

The completed emulsion (Run 91) had an average particle size of 162 nm and a nonvolatile content of approximately 30%.

Example 7

The same procedure as in Example 1 was performed except for using the following composition, changing the polymerization temperature to 65°C, and changing the emulsifier from the sodium lauryl sulfate to an ethylene oxide 20 mol adduct of polyoxyethylene (C18) ether. The HLB (NIKKOL) of the ethylene oxide 20 mol adduct of polyoxyethylene (C18) ether in Example 7 was 18.0, the molecular weight was 1150, and the cloud point was 95°C.

Monomer emulsification

Beaker 1:

Ethylene oxide 20 mol adduct of polyoxyethylene (C22) ether: 2.91 parts by weight
Ion exchanged water: 52.80 parts by weight

Beaker 2:

Methyl methacrylate: 9.61 parts by weight
Butyl acrylate: 4.95 parts by weight
Carbosiloxane dendrimer represented by General Formula (a1): 14.57 parts by weight
2-Phenoxyethanol: 0.58 parts by weight
Monomer emulsion average particle size:
Milky white, and average particle size of 194 nm.

Radical polymerization

Separable flask:

Ion exchanged water: 0 parts by weight
Monomer emulsion
3% 2,2'-Azobis[N-(2-carboxyethyl)-2-methylpropionamidine] tetrahydrate prepared with ion exchanged water: 110.8 parts by weight

Results:

No odor of methyl methacrylate or butyl acrylate was noticed.

The completed emulsion (Run 106) had an average particle size of 211 nm and a nonvolatile content of approximately 31%.

Example 8

The same procedure as in Example 1 was performed except for using the following composition, changing the polymerization temperature to 65°C, and changing the emulsifier from the sodium lauryl sulfate to a polyglyceryl monostearate (polyglyceryl-10 stearate, a reaction product of polyglycerin having 10 glycerin repeating units and stearic acid). The HLB (NIKKOL) of the polyglyceryl-10 stearate in Example 8 was 12.0, and the degree of polymerization of

the glycerin was 10.

Monomer emulsification

Beaker 1:

Polyglyceryl-10 stearate: 2.68 parts by weight
Ion exchanged water: 56.60 parts by weight

Beaker 2:

Methyl methacrylate: 8.84 parts by weight
Butyl acrylate: 4.55 parts by weight
Carbosiloxane dendrimer represented by General Formula (a1): 13.39 parts by weight
2-Phenoxyethanol: 0.54 parts by weight
Monomer emulsion average particle size:
Milky white, and average particle size of 174 nm.

Radical polymerization

Separable flask:

Ion exchanged water: 0 parts by weight
Monomer emulsion
3% 2,2'-Azobis[N-(2-carboxyethyl)-2-methylpropionamidine] tetrahydrate prepared with ion exchanged water: 100.5 parts by weight

Results:

No odor of methyl methacrylate or butyl acrylate was noticed.

The completed emulsion (Run 107) had an average particle size of 172 nm and a nonvolatile content of approximately 29%.

Example 9

The same procedure as in Example 1 was performed except for using the following composition, changing the polymerization temperature and the polymerization time to 70°C for 2 hours and 80°C for 4 hours, and changing the emulsifier from the sodium lauryl sulfate to a polyglyceryl monolaurate (polyglyceryl-10 laurate, a reaction product of polyglycerin having 10 glycerin repeating units and lauric acid). The HLB (NIKKOL) of the polyglyceryl-10 laurate in Example 9 was 15.5, and the degree of polymerization of the glycerin was 10.

Monomer emulsification

Beaker 1:

Polyglyceryl-10 laurate: 2.80 parts by weight
Ion exchanged water: 59.24 parts by weight

Beaker 2:

Methyl methacrylate: 9.25 parts by weight

Butyl acrylate: 4.77 parts by weight
Carbosiloxane dendrimer represented by General Formula (a1): 14.02 parts by weight
2-Phenoxyethanol: 0.56 parts by weight
Monomer emulsion average particle size:
Milky white, and average particle size of 150 nm.

Radical polymerization

Separable flask:

Ion exchanged water: 6.8 parts by weight
Monomer emulsion
2.5% Sodium persulfate aqueous solution prepared with ion exchanged water: 60.3 parts by weight

Results:

No odor of methyl methacrylate or butyl acrylate was noticed.

The completed emulsion (Run 103) had an average particle size of 193 nm and a nonvolatile content of approximately 30%.

Comparative Example 1

In Comparative Example 1, in the same manner as in Example 1 by using the following composition, a surfactant aqueous solution was prepared in a beaker 1, and a hydrogenation-treated decamethylpentacyclosiloxane solution containing 30 parts by weight of the component (A) polymer formed from radical polymerization of 40 parts by weight of carbosiloxane dendrimer of the component (a1) and the total of 60 parts by weight of component (a2) (weight ratio of methyl methacrylate to butyl acrylate of 11:1) was prepared in a beaker 2. The polymer solution in the beaker 2 was added to the beaker 1 and agitated for a several minutes, and then passed through a pressure of 400 to 500 kg/cm2 for multiple times by using a homogenizer to obtain an emulsion (Run A) having a solid content of 40% and an average particle size of 469 nm. During the emulsification, a trace amount polymer deposition also occurred. The HLB (NIKKOL) of the ethylene oxide 9 mol adduct of polyoxyethylene (C12-14) ether in Comparative Example 1 was 13.5, the molecular weight was 596, and the cloud point was 59°C.

Beaker 1:

Ethylene oxide 9 mol adduct of polyoxyethylene (C12-14) ether: 0.80 parts by weight
Ion exchanged water: 59.20 parts by weight

Beaker 2:

Decamethylpentacyclosiloxane solution containing 30 parts by weight of the component (A): 40.00 parts by weight

Comparative Example 2

The same procedure as in Comparative Example 1 was performed except for using the following composition. The HLB (NIKKOL) of the ethylene oxide 15 mol adduct of polyoxyethylene (C13) ether in Comparative Example 2 was 15.5, the molecular weight was 860, and the cloud point was 100°C.

Beaker 1:

Ethylene oxide 9 mol adduct of polyoxyethylene (C12-14) ether: 0.80 parts by weight
Ion exchanged water: 69.20 parts by weight

Beaker 2:

Hydrogenation-treated dodecamethylpentasiloxane solution containing 30 parts by weight of the component (A) polymer formed from radical polymerization of 50 parts by weight of carbosiloxane dendrimer of the component (a1) and the total of 50 parts by weight of component (a2) (weight ratio of methyl methacrylate to butyl acrylate of 3:1): 30 parts by weight

Results:

An emulsion (Run B) having a solid content of 30% and an average particle size of 533 nm was obtained. During the emulsification, a trace amount polymer deposition also occurred. The component (A) polymer had a weight average molecular weight of 40000 measured by gel permeation chromatography by using toluene as a solvent and based on calibration with polystyrene.

Comparative Example 3

Monomer emulsification

In a beaker 1, 3.00 parts by weight of polyoxyethylene (C13) ether EO 15 mol was weighed, and then 48.35 parts by weight of ion exchanged water was added and agitated to form an aqueous solution. In a beaker 2, 9.90 parts by weight of methyl methacrylate, 5.10 parts by weight of butyl acrylate, and 15.00 parts by weight of carbosiloxane dendrimer represented by General Formula (1), and 0.60 parts by weight of 2-phenoxyethanol were added and homogenized. After the mixture in the beaker 2 was added to the beaker 1 and then agitated for several minutes, it was passed through a pressure of 400 to 500 kg/cm2 for multiple times by using a homogenizer to obtain a milky white monomer emulsion having an average particle size of 112 nm.

Radical polymerization

In a separable flask, 2.98 parts by weight of ion exchanged water and a part of the obtained monomer emulsion were charged, and the temperature was raised to 70°C while the mixture was agitated. However, when the liquid temperature reached 59°C, the emulsion appearance in the flask was changed from white to semitransparent. Emulsion breakage was confirmed. The same operation was performed again for the confirmation, and then the emulsion breakage was confirmed at approximately 59°C. Therefore, it was determined that radical polymerization was not possible.

The evaluation method of the cosmetic composition was described below.

Average particle size measurement

By using a laser diffraction particle size distribution measurement device (available from Coulter; trade name: N4 MD), the average particle size (the value of the particle size corresponding to the size at 50% of accumulated particle distribution) was measured.

Solid content measurement

The solid content was determined as follows. Approximately 2 g of a sample was placed on an aluminum plate and then dried for 2 hours in an oven at 105°C. The solid content was obtained as a value of the amount of the residue divided by the weight before the drying.

Waterproofness evaluation

The obtained emulsion was thinly coated on a glass plate (alkali-treated windshield having a width of 26 mm * a length of 76 mm) by using a Meyer bar and air-dried at room temperature for 3 days. Thereafter, 10 water droplets were placed on the surface thereof to measure the contact angle relative to water, and the average of the 10 values was used as the result. As the measurement device, the KRUSS contact angle meter (DSA Mk2) was used.

Sebum resistance evaluation

The same procedure as in waterproofness evaluation was performed except for using an artificial sebum in place of the water droplet to measure a contact angle.

Transmittance measurement

Dilution to 0.2% was performed by using ion exchanged water, and the transmittance at a wavelength of 580 nm was measured by a spectrophotometer CM-5 (available from Konica Minolta, Inc.) by using a transmission cell having a width of 10 mm. (Transmittance of ion exchanged water was set as a value of 100)

[Table 1]

| | | Example | | | | | | | | |
|---|---|---|---|---|---|---|---|---|---|---|
| | | **1** | **2** | **3** | **4** | **5** | **6** | **7** | **8** | **9** |
| | | RUN ***41*** | RUN ***52*** | RUN ***53*** | RUN ***55*** | RUN ***36*** | RUN ***91*** | RUN ***106*** | RUN ***107*** | RUN ***103*** |
| Emulsion portion | Sodium lauryl sulfate | 0.53 | | | | | | | | |
| | Laureth-1 phosphate (90%) | | 0.67 | | 0.68 | | | | | |
| | Sodium lauryl phosphate | | | 0.60 | | | | | | |
| | Polyoxyethylene (C16) ether EO 20 mol | | | | | | 2.90 | | | |
| | Polyoxyethylene (C18) ether EO 20 mol | | | | | | | 2.91 | | |
| | Polyglyceryl-10 stearate | | | | | | | | 2.68 | |
| | Polyglyceryl-10 laurate | | | | | | | | | 2.80 |
| | - | - | - | - | - | - | - | - | - | - |
| | Reactive emulsion A* | | | | | 1.91 | | | | |
| | Reactive emulsion B* | | | | | 0.46 | | | | |
| | Water | 51.72 | 46.87 | 49.72 | 47.48 | 37.34 | 52.55 | 52.80 | 56.60 | 59.24 |
| | 50% Sodium hydroxide | | 0.17 | | 0.17 | | | | | |
| | 2-Phenoxyethanol | | 0.30 | 0.30 | 0.31 | | 0.58 | 0.58 | 0.54 | 0.56 |

| | | | | | | | | | | |
|---|---|---|---|---|---|---|---|---|---|---|
| | (a2) Methyl methacrylate | 10.09 | 11.49 | 11.45 | 18.63 | 10.99 | 9.57 | 9.61 | 8.84 | 9.25 |
| | (a2) Butyl acrylate | 3.18 | 3.61 | 3.62 | 5.86 | 3.45 | 4.93 | 4.95 | 4.55 | 4.77 |
| | (a1) Carbosiloxane dendrimer | 13.23 | 15.06 | 15.07 | 6.10 | 14.36 | 14.50 | 14.57 | 13.39 | 14.02 |
| Drop portion | Water (for flask) | 8.00 | 6.75 | 4.17 | 5.47 | 17.11 | 0.47 | 0 | 0 | 0 |
| | Water (for initiator) | 13.12 | 14.93 | 14.92 | 15.14 | 14.25 | 14.28 | 14.10 | 12.99 | 8.2 |
| | Potassium persulfate | 0.13 | 0.15 | 0.15 | 0.15 | 0.14 | 0.22 | | | |
| | Sodium persulfate | | | | | | | | | 0.21 |
| | 2,2'-Azobis[N-(2-carboxyethyl)-2-methylpropionamidine] tetrahydrate | | | | | | | 0.44 | 0.40 | |
| Average particle size during monomer emulsification (nm) | | 153 | 117 | 125 | 82 | 170 | 121 | 194 | 174 | 150 |
| Average particle size after polymerization (nm) | | 143 | 114 | 109 | 105 | 174 | 162 | 211 | 172 | 193 |
| Carbosiloxane dendrimer in component (A) polymer wt.% | | 50 | 50 | 50 | 20 | 50 | 50 | 50 | 50 | 50 |
| Component (A) polymer in emulsified particle wt.% | | 100 | 100 | 100 | 100 | 100 | 100 | 100 | 100 | 100 |
| Solid content (wt.%) | | 30 | 30 | 30 | 30 | 30 | 30 | 31 | 29 | 30 |
| Transmittance (%) | | 82.4 | 84.5 | 86.5 | 81.6 | 70.1 | 71.9 | 64.7 | 63.4 | 52.8 |
| Water contact angle (°) | | 107.0 | 115.6 | 113.5 | 131.4 | 102.3 | 108.8 | 121.4 | 117.4 | 116.2 |
| Contact angle by using artificial sebum (°) | | 65.4 | 77.2 | 67.7 | 56.4 | 70.1 | 79.7 | 91.3 | 82.4 | 80.7 |

[Table 2]

| | | Comparative Example | | |
|---|---|---|---|---|
| | | 1 | 2 | 3 |
| | | RUN **A** | RUN **B** | - |
| Emulsion portion | Polyoxyethylene (C12-14) ether EO 9 mol | 0.80 | 0.80 | |
| | Polyoxyethylene (C13) ether EO 15 mol | | | 3.00 |
| | Water | 59.20 | 69.20 | 48.35 |
| | 2-Phenoxyethanol | | | 0.60 |
| | (a2) Methyl methacrylate | 6.60 | 3.42 | 9.90 |
| | (a2) Butyl acrylate | 0.60 | 1.08 | 5.10 |
| | (a1) Carbosiloxane dendrimer | 4.80 | 4.50 | 15.00 |
| | Decamethylpentacyclosiloxane | 28.00 | | |
| | Dodecamethylpentasiloxane | | 21.00 | |
| Drop portion | Water (for flask) | | | 2.98 |
| | Water (for initiator) | | | 14.77 |
| | Potassium persulfate | | | 0.23 |
| Average particle size during monomer emulsification (nm) | | 469 | 533 | 112 |
| Average particle size after polymerization (nm) | | 469 | 533 | Unmeasurable |
| Carbosiloxane dendrimer in component (A) polymer wt.% | | 40 | 50 | |
| Component (A) polymer in emulsified particle wt.% | | 30 | 30 | |
| Solid content (wt.%) | | 40 | 40 | |
| Transmittance (%) | | 16.1 | 13.8 | |
| Water contact angle (°) | | 89.6 | 88.3 | |
| Contact angle by using artificial sebum (°) | | 49.2 | 49.8 | |

* Reactive emulsifier A: oxirane polyaddition product (65%) of {reaction product of alkanol (C = 10 to 14, branched) and 1-(allyloxy)-2,3-epoxypropane} containing {α-[2-(allyloxy)-1-({[alkyl (C = 10 to 14)]oxy}methyl)ethyl]-ω-hydroxypoly(n = 1 to 100)(oxyethylene)} as a main component; cloud point: 100°C
* Reactive emulsifier B: alkenyl potassium succinate (30%)

To measure the film physical properties, the contact angle value was measured as the indicator. The small contact angle increases the contact area between the film and water or sebum, leading to fragility of the film such as swelling. The large contact angle decreases the contact area, and thus the effect on the film is small. Exhibition of a large contact angle value indicates high long-lasting characteristics of waterproofness and sebum resistance characteristics. Furthermore, as a matter of course, rub resistance and adhesion (durability) to hair become superior. Examples 1 to 9 had contact angles of 100° or greater for the water contact angle, and had 50° or greater for the artificial sebum. Furthermore, because Comparative Examples 1 and 2 were emulsions obtained by emulsifying polymer solutions although the emulsions contained the carbosiloxane dendrimer components that were the same as those in Examples, Comparative Examples 1 and 2 includes the solvent in the oil and thus the volume average particle size of the emulsion particles became large, the content of the polymer in the emulsified particles became low, and it was difficult to increase the content of the polymer further.

The evaluation test results of performances of the film formed as described above are described below.

The results obtained by using the film forming agent (A) which was the emulsion produced in Example 2 (RUN 52),

the film forming agent (B) which was the emulsion produced by the same method as in Comparative Example 2 while

the polymer wt.% was adjusted to 40 wt.% (RUN-B),
and the film forming agent (C) which was 100 % silicone resin (trimethylsiloxy silicic acid).

Note that the solvent used in the dilution was purified water for (A), dimethicone 2 cs for (B), and isododecane for (C).

Softness evaluation of film

A film sample was produced on a rubber sheet formed from silicone. The film sample was produced by diluting each of (A), (B), and (C) having a solid content of 20 wt.% with a diluent, coating the diluted solution to the thickness of 50 μm, and drying the coating overnight at room temperature. Thereafter, appearance after the film sample was stretched to 150% and then restored back was observed. As a result, the film samples of (A) and (B) each maintained a uniform film with no cracks on the surface even after being stretched; however, many cracks occurred on the film sample of (C). Thus, it was shown that (A) and (B) had superior softness of the film compared to the softness of the (C).

Film integrity evaluation

A film sample was formed on a collagen film, and the permeated amount of blue dye was evaluated (the graph below shows the concentration of the dye after 1 hour and after 6 hours). The film sample was produced by coating the diluted solution having a solid content of 20 wt.% to the thickness of 50 μm, and drying the coating overnight at room temperature. It was shown that (A) and (B) had high integrity of the film because (A) and (B) had significantly smaller permeated amount of the dye compared to the case of the (C).

[Table 3]

| | 0 (hr) | 1 (hr) | 6 (hr) |
|---|---|---|---|
| (A) (wt.%) | 0 | 0.04 | 0.17 |
| (B) (wt.%) | 0 | 0.70 | 8.8 |
| (C) (wt.%) | 0 | 34.8 | 71.8 |

Sebum resistance evaluation

A film sample (VITRO-SKIN(R)) was produced on a glass microscope slide. Water repellency of each of the film forming agents (A) to (C) was evaluated (the values below show the contact angle with water after 5 seconds and after 115 seconds). The film sample was produced by coating the diluted solution having a solid content of 20 wt.% to the thickness of 50 μm, and drying the coating overnight at room temperature. The mean value and the standard deviation of the contact angle were obtained.

[Table 4]

| | at 0 (sec) | | at 115 (sec) | |
|---|---|---|---|---|
| | Mean | St.Dev. | Mean | St.Dev. |
| Baycusan C1000(*) | 28.5 | 1.1 | 10.9 | 0.2 |
| (C) | 51.7 | 0.5 | 30.3 | 1.3 |
| (B) | 54.7 | 3.1 | 53.9 | 2.9 |
| (A) | 77.8 | 2.9 | 70.2 | 1.2 |
| * Baycusan C1000: Aqueous polyurethane dispersion, available from Covestro Deutschland AG | | | | |

Although the film forming agent (A) contained a surfactant in the composition, the film forming agent (A) exhibited higher sebum resistance compared to the sebum resistance of the (B) which contained no surfactant.

Examples of cosmetics

The film forming agent (A) was mixed as described in the composition of Table 5, and degree of precipitation was measured after 15 minutes.

[Table 5]

| Component | control | pH 3 | pH 12 | Glycerin 10% | Prop Gly 50% | NaCl 0.5% | NaCl 3% | $Mg_2SO_4$ 0.5% |
|---|---|---|---|---|---|---|---|---|
| Film forming agent (A) | 3 | 3 | 3 | 3 | 3 | 3 | 3 | 3 |
| Citric acid (25%) | - | 0.12 | - | - | - | - | - | - |
| NaOH (25%) | - | - | 0.11 | - | - | - | - | - |
| Glycerin | - | - | - | 1.5 | - | - | - | - |
| Propylene glycol | - | - | - | - | 6 | - | - | - |
| NaCl | - | - | - | - | - | 0.075 | 0.45 | |
| $Mg_2SO_4$ | - | - | - | - | - | - | - | 0.075 |
| Water | 12 | 11 | 10 | 10.5 | 6 | 11.925 | 11.55 | 11.925 |
| Degree of precipitation | 0.03 | 0.03 | 0.05 | 0.07 | 0.09 | 0.01 | 14.46 | 19.29 |

[Formulation Example 1]

O/W Sunscreen

| Composition | wt.% |
|---|---|
| 1. Titanium oxide dispersion (average particle size: 0.1 μm) | 15 |
| 2. Ethylhexyl methoxycinnamate | 5 |
| 3. PEG-60 hydrogenated castor oil | 2 |
| 4. Glycerin | 6 |
| 5. Ethanol | 5 |
| 6. Carbomer 2% aqueous solution | 15 |
| 7. Sodium hydroxide 1% aqueous solution | 15.75 |
| 8. Purified water | Balance |
| 9. Acrylic emulsion of Example 1 (Run 41) | 10 |

Production method

1. Components 1 and 2 were mixed. (Mixture 1)

2. Components 3 to 8 were mixed until the mixture was homogenized. (Mixture 2)

3. While the Mixture 2 was agitated, Mixture 1 was gradually added to emulsify. (Emulsion 3)

4. Component 9 was added to Emulsion 3 and mixed.

[Formulation Example 2] O/W Sunscreen spray

| Composition | wt.% |
|---|---|
| 1. PEG-12 dimethicone * 1 | 3.5 |
| 2. Lauryl PEG/PPG - 18/18 methicone *2 | 0.5 |
| 3. Caprylyl methicone *3 | 1 |
| 4. Phenyl trimethicone *4 | 1 |
| 5. Mixture of ethylhexyl methoxycinnamate and diethylamino hydroxybenzoyl hexyl benzoate *5 | 6 |
| 6. Trilaureth-4 phosphate *6 | 0.1 |
| 7. Bis-PEG-18 methylether dimethylsilane *7 | 2 |
| 8. Ethanol | 10 |
| 9. Sodium dihydrogenphosphate | 0.01 |
| 10. Sodium hydrogenphosphate | 0.01 |
| 11. Antiseptic | q.s. |
| 12. Purified water | Balance |
| 13. 1,3-Butylene glycol | 5 |
| 14. Purified water | 5 |
| 15. Phenylbenzimidazole sulfonic acid *8 | 3 |
| 16. Triethanolamine | 2 |
| 17. Acrylic emulsion of Example 3 (Run 53) | 10 |

*1 XIAMETER(R) OFX-5329 Fluid, available from Dow Corning Toray Co., Ltd.
*2 Dow Corning(R) 5200 Formulation Aid, available from Dow Corning Toray Co., Ltd.
*3 Dow Corning(R) FZ-3196, available from Dow Corning Toray Co., Ltd.
*4 Dow Corning Toray SH556, available from Dow Corning Toray Co., Ltd.
*5 Uvinul A Plus B, available from BASF
*6 Hostaphat KL340D, available from Clariant
*7 Dow Corning(R) 2501 Cosmetic Wax, available from Dow Corning Toray Co., Ltd.
*8 PARSOL HS, available from DSM Nutrition Japan KK

Production method

1. Components 1 to 8 were mixed. (Mixture 1)

2. Components 9 to 13 were mixed. (Mixture 2)

3. While the Mixture 2 was agitated, Mixture 1 was gradually added to emulsify. (Emulsion 3)

4. Components 14 to 16 were mixed and added to Emulsion 3. (Mixture 4)

5. Component 17 was added to Mixture 4 and agitated.

[Formulation Example 3] Mascara

| Composition | wt.% |
|---|---|
| 1. Beeswax | 10 |
| 2. Ozokerite | 7 |
| 3. Carnauba wax | 3 |
| 4. Stearic acid | 5 |
| 5. Glyceryl stearate | 5 |
| 6. Antiseptic | q.s. |
| 7. Purified water | Balance |

(continued)

| Composition | wt.% |
|---|---|
| 8. Propylene glycol | 5 |
| 9. Triethanolamine | 1.5 |
| 10. Black oxide of iron | 10 |
| 11. Acrylic emulsion of Example 4 (Run 55) | 15 |

Production method

1. Components 1 to 6 were mixed and heated to 85°C to produce Mixture 1.

2. Components 7 to 9 were mixed and heated to 80°C to produce Mixture 2.

3. While the Mixture 2 was agitated, Mixture 1 was added to emulsify. (Emulsion 3)

4. Emulsion 3 was added to Component 10, and the mixture was cooled to 50°C while being agitated, and Component 11 was added thereto and agitated.

[Formulation Example 4] O/W Sunscreen

| Composition | wt.% |
|---|---|
| 1. Silicone emulsion premix *1 | 10 |
| 2. Trilaureth-4 phosphate *2 | 0.05 |
| 3. Ethylhexyl methoxycinnamate | 8.5 |
| 4. Diethylamino hydroxybenzoyl hexyl benzoate | 1.5 |
| 5. Dimethicone | 2 |
| 6. Carbinol-modified silicone *3 | 1 |
| 7. Phenyl-modified silicone *4 | 2 |
| 8. Carbomer 2% aqueous solution *5 | 22.5 |
| 9. Purified water | Balance |
| 10. Na hydroxide 1% aqueous solution | q.s. |
| 11. PPG-10 methylglucose *6 | 0.4 |
| 12. Ethanol | 2 |
| 13. 1,3-Butylene glycol | 5 |
| 14. Glycerin | 5 |
| 15. Antiseptic | q.s. |
| 16. Acrylic emulsion of Example 1 (Run 41) | 7 |

*1 FB-2540 Emulsifier Blend, available from Dow Corning Toray Co., Ltd.
*2 Hostaphat KL340D, available from Clariant
*3 Dow Corning(R) 5562 Carbinol Fluid, available from Dow Corning Toray Co., Ltd.
*4 Dow Corning Toray SH556, available from Dow Corning Toray Co., Ltd.
*5 Carbopol(R) Ultrez10 Polymer, available from The Lubrizol Corporation
*6 MACBIOBRIDE MG-10P, available from NOF Corporation

Production method

1. Components 1 to 7 were mixed. (Mixture 1)

2. Components 8 to 10 were mixed. (Mixture 2)

3. While the Mixture 2 was agitated, Mixture 1 was gradually added to emulsify. (Emulsion 3)

4. Components 11 to 16 were mixed. (Mixture 4)

5. Mixture 4 was added to Emulsion 3 and mixed.

[Formulation Example 5] O/W Sunscreen (hydrophilic microparticle)

[Table 6]

| | |
|---|---|
| Phase A (wt.%) | |
| PEG-12 dimethicone *1 | 3.5 |
| Lauryl PEG/PPG - 18/18 methicone *2 | 0.5 |
| Caprylyl methicone * | 1 |
| Phenyl trimethicone *4 | 1 |
| Ethylhexyl methoxycinnamate | 6.4 |
| Diethylamino hydroxybenzoyl hexyl benzoate | 3.6 |
| Octocrylene | 2 |
| Ethylhexyl salicylate | 3 |
| Trilaureth-4 phosphate | 0.1 |
| Bis-PEG-18 methylether dimethylsilane *5 | 2 |
| Alcohol | 10 |
| Phase B (wt.%) | |
| Disodium phosphate | 0.01 |
| Sodium phosphate | 0.01 |
| Phenoxyethanol (and) ethylhexylglycerin | 0.5 |
| Purified water | 36.43 |
| Butylene glycol | 5 |
| Carbomer *6 | 0.45 |
| 1% NaOH | 10.5 |
| Phase C (wt.%) | |
| Film forming agent (A) | 4 |
| Hydrophilic microparticle | 10 |
| *1 Dow Corning Toray(R) SS-2804 (Dow Corning Toray Co., Ltd.)<br>*2 Dow Corning(R) 5200 Formulation Aid (Dow Corning Toray Co., Ltd.)<br>*3 Dow Corning(R) FZ-3196 (Dow Corning Toray Co., Ltd.)<br>*4 Dow Corning(R) 556 Cosmetic Fluid (Dow Corning Toray Co., Ltd.)<br>*5 Dow Corning(R) 2501 Cosmetic Wax (Dow Corning Toray Co., Ltd.)<br>*6: Carbomer: Any of Carbopol(R) 980, Carbopol(R) 2984, Carbopol(R) 1382, Carbopol(R) 981, Carbopol(R) Ultrez 10, Carbopol(R) ETD 2050, and other. | |

Preparation method:

The components of Phase A were mixed until the components was homogenized.

The components of Phase B were mixed until the components was homogenized.
The components of Phase A were mixed into the Phase B while being mixed.
Phase C was added to Phase AB and mixed.

Cosmetic Material Evaluation Example 1

O/W sunscreen cream

For emulsions produced based on Examples 1 to 9 and Comparative Examples 1 and 2, O/W (oil-in-water) sunscreen creams having the compositions listed in Table 2 below were produced. The O/W sunscreen creams were subjected to evaluations of (1) blending stability and (2) contact angle with water by the following methods, and the results are also listed in Table 2. Note that O/W refers to water-in-oil type. The compounded amount used in the composition refers to part by weight of the case where the amount of the final product emulsion is assigned a value of 100 parts by weight, unless otherwise noted. Furthermore, in the case where the compounded amount is described as "balance", the balance refers to the amount obtained by subtracting part by weight of the other components from the part by weight of the final product.

Composition of O/W sunscreen cream used for evaluation

| | |
|---|---|
| Phase A: Polysorbate 80*1 | 1 |
| Mineral oil *2 | 10 |
| Triethylhexanoin | 5 |
| Diethylamino hydroxybenzoyl hexyl benzoate | 2.5 |
| Ethylhexyl methoxycinnamate | 7.5 |
| Caprylyl methicone | 10 |
| Phase B: Titanium oxide dispersion *3 | 10 |
| Carbomer 2% aqueous solution *4 | 15 |
| Purified water | Balance |
| Sodium hydroxide 1% aqueous solution | 15.75 |
| 1,3-Butylene glycol | 5 |
| Glycerin | 2 |

*1 RHEODOL TW-0120V, available from Kao Corporation
*2 HICALL K-230, available from Kaneda Co., Ltd.
*3 Microparticulate titanium oxide dispersion (particle size of dispersoid: 0.1 μm) 40 wt.%, cyclopentasiloxane 50 wt.%, ES-5600 Silicone Glycerol Emulsifier 10 wt.%, available from Tayca Corporation
*4 Carbopol 940

Preparation method

Each of Phase A and Phase B was agitated until each of Phase A and Phase B was homogenized.
While being agitated, the solution of Phase B was gradually added to the solution of Phase A to emulsify. (Emulsion 2)
Emulsion 2 was added to 10 parts of the emulsion solution obtained in each of Examples 1 to 9 and Comparative Examples 1 and 2, or to 10 parts of water in place of the emulsion solution, to make the entire mixture 100 parts by weight, and agitated until the mixture was homogenized.

Evaluation (1) evaluation method for blending stability

The obtained emulsion was observed by using a transmission microscope on the next day or after being left in an oven at 50°C for 1 month, to confirm the change over time of the emulsion droplets.

Evaluation

Good: Almost no change in the emulsion droplets was observed.
Poor: Emulsion droplets were merged together.

Evaluation (2) evaluation method for contact angle with water

The obtained emulsion was coated uniformly on a glass microscope slide by using a bar coater No. 5 and dried for 1 day at room temperature. Thereafter, 10 water droplets were placed on the surface thereof to measure the contact angle relative to water, and the average of the 10 values was used as the result. As the measurement device, the KRUSS contact angle meter (DSA Mk2) was used.

[Table 7]

| | Example | | | | | | | | | Comparative Example | | |
|---|---|---|---|---|---|---|---|---|---|---|---|---|
| Component | 1(Run41) | 2(Run52) | 3(Run53) | 4(Run55) | 5(Run36) | 6(Run91) | 7(Run106) | 8(Run107) | 9(Run103) | Run A | Run B | Water |
| Blending stability | Good | Good | Good | Good | Good | Good | Good | Good | Good | Poor | Poor | Good |
| Contact angle with water | 63 | 57 | 52 | 41 | 63 | - | - | - | - | 23 | 25 | 23 |

Cosmetic Material Evaluation Example 2

Long-lasting characteristic (rub resistance) evaluation of mascara formulation

A mascara containing a film forming agent (A) and a Control (sample containing no film forming agent (A)) for comparison were coated on a PMMA plate that simulated skin. Long-lasting characteristic was evaluated by observing a change in color tone of the coating before and after rubbing the top of the coating with a felt material.

The mascara prepared by the following formulation was coated on the PMMA plate that simulated skin to the thickness of 25 $\mu$m, and the initial brightness (L value) was measured by using a color meter (BYK Gardner Spectro-Guide: 6801 Color Spectrophotometer). Thereafter, after 100 $\mu$L of water was brought into contact with the coated surface for 1 minute, the mascara was rubbed one time with a probe formed from a felt base material, and then the L value was measured by using a color meter. The difference of the L value of the graph indicates the value before and after the rubbing with felt.

[Table 8]

| | Brightness (L value) | Standard deviation |
|---|---|---|
| Control | 10.89 | 0.63 |
| (A) | 4.36 | 1.68 |

Mascara formulation used for evaluation

| | |
|---|---|
| Phase A | |
| 1. Beeswax *1 | 9.5 |
| 2. Stearyl dimethicone *2 | 6.5 |
| 3. Carnauba wax *3 | 2.8 |
| 4. Stearic acid *4 | 4.8 |

(continued)

| | |
|---|---|
| Phase A | |
| 5. Glyceryl stearate *5 | 4.8 |
| Phase B | |
| 1. Water | Balance |
| 2. PG | 4.8 |
| 3. Triethanolamine 85% | 1.4 |
| Phase C | |
| 1. Black oxide of iron *6 | 4.8 |
| Phase D | |
| 1. Film forming agent (A) | 13.3 |
| 2. Isododecane | 18.0 |

*1: Kahlwax 8105, available from Kahlwax
*2: 2503 Cosmetic Wax, available from Dow Corning Corporation
*3: Kahlwax 2442, available from Kahlwax
*4: Stearic acid, available from Lipo Chemicals
*5: Cutina(R) GMS, available from BASF
*6: UNIPURE LC 989 AS EM, available from Sensient Cosmetic Technologies

Preparation method

1. Phase A was mixed and heated to 85°C.

2. Phase B was mixed and heated to 80°C.

3. While Phase A was being mixed, Phase B was added.

4. While Phases AB were being mixed, Phase C was added.

5. The mixture was cooled to 35°C.

6. Phase D was added to Phase ABC.

7. The mixture was cooled to room temperature while being mixed.

Cosmetic Material Evaluation Example 3

Long-lasting characteristic (rub resistance) evaluation of lipstick formulation

A lipstick containing a film forming agent (A) and a Control (sample containing no film forming agent (A)) for comparison were coated on a PMMA plate that simulated skin. Long-lasting characteristic was evaluated by observing a change in color tone ΔE of the coating by rubbing the top of the coating with a felt material. The lipstick prepared by the following formulation was coated on the PMMA plate that simulated skin to the thickness of 25 μm, and the lipstick was rubbed with a probe formed from a felt base material. Then, ΔE value was measured by the color meter described above, and this operation was repeated for 10 times (black line of the graph). Furthermore, a sample having a coated surface which was brought into contact with 100 μL of olive oil for 1 minute was also rubbed with a probe in the same manner, and then the ΔE thereof was measured. The sample which was brought into contact with the oil was shown as "oil", and the sample which was not brought into contact with the oil was recorded as "no oil".

Lipstick formulation used for evaluation

| | |
|---|---|
| Phase A | |
| 1. Silicone crosslinked material *1 | 25.0 |
| 2. Isododecane | 15.0 |
| 3. Silica silylate*2 | 0.1 |
| 4. Silicone emulsion *3 | 2.5 |
| 5. Pigment dispersion *4 | 10.0 |
| Phase B | |
| 6. Water | Balance |
| 7. Propanediol | 10.0 |
| 8. Film forming agent (A) | 10.0 |
| 9. Antibacterial-antiseptic agent *5 | 0.5 |
| 10. Antibacterial-moisturizing agent *6 | 0.5 |

*1: EL-7040 Hydro Elastomer Blend, available from Dow Corning Corporation
*2: VM-2270 Aerogel Fine Particles, available from Dow Corning Corporation
*3: BY 25-337, available from Dow Corning Corporation
*4: Covanol OS3703, available from Sensient Cosmetics Technologies
*5: Symsave(R) H, available from Symrise AG
*6: Symsave(R) 68, available from Symrise AG

Preparation method

1. Phase A was mixed until Phase A was homogenized.
2. While Phase A was being agitated, Phase B was added.

Evaluation of wrinkle concealing effect

A wrinkle concealing cosmetic material was prepared by using the film forming agent (A) based on the following formulation example, and evaluation of wrinkle concealing effect was performed by using a skin imaging device (Visia(R)-CR, available from Canfield Scientific) (in a condition at room temperature of 20°C and a humidity of 50%). Around the outer corner of an eye of face, the wrinkle cosmetic material containing a film forming agent (A) was applied at the amount of 2 mg per 1 $cm^2$, and photographs of skin surface after 15 minutes and after 1 hour and a photograph of skin to which application was not carried out were taken. When observation was performed by a panel of 12 members, all the members of the panel answered that the skin to which the film forming agent (A) was applied had less wrinkles, it was shown that the film forming agent (A) exhibited wrinkle concealing effect.

Formulation of wrinkle cosmetic material hydrogel base material used for evaluation

| | |
|---|---|
| Phase A | |
| 1. Carbopol(R) Ultrez 21 Polymer (available from Lubrizol) 0.5 | |
| 2. Dissolvine(R) Na2-P (available from Akxo Nobel) | 0.05 |
| 3. Triethanolamine | 0.4 |
| 4. Water | Balance |
| Phase B | |
| 1. Creasil(R) ID CG (available from The Innovation Company) | 5.0 |
| 2. Film forming agent (A) | 0.5 - 10.0 |
| 3. Euxyl (R) PE9010 (available from Schulke & Mayr) | 0.5 |
| 4. Propylene Glycol (available from The Dow Chemical Company) | 3.0 |

Preparation method:

After Phase A was mixed, Phase B was mixed thereto to mix the Phase A and Phase B.

Cosmetic Material Evaluation Example 4

O/W foundation cream

For emulsions produced based on Examples 1 to 9 and Comparative Examples 1 and 2, O/W (oil-in-water) sunscreen creams having the compositions listed in Table 2 below were produced. The O/W sunscreen creams were subjected to evaluation of blending stability by the following method, and the results are listed together in Table 9.

The compounded amount used in the composition refers to part by weight of the case where the amount of the final product emulsion is assigned a value of 100 parts by weight, unless otherwise noted. Furthermore, in the case where the compounded amount is described as "balance", the balance refers to the amount obtained by subtracting part by weight of the other components from the part by weight of the final product.

Formulation of O/W foundation used for evaluation

| | |
|---|---|
| Phase A | |
| 1. Stearic acid | 1 |
| 2. Polysorbate 80*1 | 1.2 |
| 3. Sesquioxane sorbitan *2 | 0.2 |
| 4. Glyceryl stearate *3 | 1.5 |
| 5. Behenyl alcohol *4 | 2.5 |
| 6. Dimethicone 2cs | 8 |
| 7. Dimethicone 5cs | 3 |
| 8. Squalane | 3 |
| 9. Isotridecyl isononanoate *5 | 3 |
| Phase B | |
| 10. Tri(caprylic acid/capric acid)glyceride | 3 |
| 11. Sorbitan sesquiisostearate *6 | 0.5 |
| 12. Pigment-grade titanium oxide *7 | 8.5 |
| 13. Yellow oxide of iron *8 | 0.4 |
| 14. Red oxide of iron *9 | 1 |
| 15. Black oxide of iron *10 | 0.1 |
| Phase C | |
| 16. Purified water | Balance |
| 17. Carbomer 2% aqueous solution *11 | 10 |
| 18. 1,3-Butylene glycol | 8 |
| 19. Sodium hydroxide 1% aqueous solution | 15 |
| Phase D | |
| 20. Acrylic emulsion of examples and comparative examples | 10 |

*1: RHEODOL TWD-130V, available from Kao Corporation
*2: EMALEX SPO-150, available from Nihon Emulsion Co., Ltd.
*3: RHEODOL MS-50, available from Kao Corporation
*4: Behenyl alcohol, available from Nikko Chemicals Co., Ltd.
*5: EMALEX INTD-139, available from Nihon Emulsion Co., Ltd.
*6: NIKKOL SI-15RV, available from Nikko Chemicals Co., Ltd.
*7: SI titanium CR-50, available from Miyoshi Kasei, Inc.
*8: SA yellow, available from Miyoshi Kasei, Inc.
*9: SA iron red, available from Miyoshi Kasei, Inc.
*10: SA black, available from Miyoshi Kasei, Inc.
*11: Carbopol 940

Production method

1. Phase A was mixed and heated to 70°C.

2. Phase B was mixed by using three rolls until Phase B was homogenized.

3. Phase B was added to Phase A and mixed.

4. Phase C was mixed and heated to 70°C.

5. While Phase C was being agitated, Phase AB was gradually added and emulsified, and cooled to 50°C or lower while being agitated.

6. Component 20 was added to the emulsion and agitated.

Evaluation method of blending stability

The obtained emulsion was observed by using a transmission microscope on the next day or after being left in an oven at 50°C for 2 weeks, to confirm the change over time of the emulsion droplets.

Evaluation

Good: Almost no change in the emulsion droplets was observed.

Poor: Emulsion droplets were merged together.

[Table 9]

| | Example | | | | | | | | | Comparative Example | | |
|---|---|---|---|---|---|---|---|---|---|---|---|---|
| Component | 1(Run41) | 2(Run52) | 3(Run53) | 4(Run55) | 5(Run36) | 6(Run91) | 7(Run106) | 8(Run107) | 9(Run103) | Run A | Run B | Water |
| Blending stability | Good | Good | Good | Good | Good | Good | Good | Good | Good | Poor | Poor | Good |

[INDUSTRIAL APPLICABILITY]

An embodiment of the present invention can be applied in cosmetic compositions and cosmetics as a film forming agent but is not limited thereto and can be used in other applications and products. That is, an embodiment of the present invention can be employed in applications where water repellency effects, oil repellency effects, integrity, strength, rub resistance, and/or softness are required for a film, without limitations regarding the fields. In particular, an embodiment of the present invention is employed in coating compositions, water repellency-imparting agents for fibers, treatment agents or protective agents for inorganic materials/organic materials, and the like. Furthermore, because the film forming agent of an embodiment of the present invention can be used in an aqueous environment, an embodiment of the present invention is employed as a main agent or an additive in an aqueous composition.

**Claims**

**1.** A cosmetic composition comprising at least:

(A) 100 parts by weight of at least one vinyl polymer having at least one carbosiloxane dendrimer structure in a side chain;
(B) from 0.1 to 30 parts by weight of a surfactant; and
(X) water;
wherein an emulsified particle comprising at least the component (A) and the component (B) is in an aqueous phase; and a weight average molecular weight of the component (A) is at least 80000.

**2.** The cosmetic composition according to claim 1, wherein the component (A) constitutes not less than 50 wt.% of an oil phase in the emulsified particle.

**3.** The cosmetic composition according to claim 2, wherein the cosmetic composition is for skin or for hair.

**4.** The cosmetic composition according to claim 1 or 2, wherein the surfactant of the component (B) is an ionic surfactant and/or a nonionic surfactant.

**5.** The cosmetic composition according to any one of claims 1 to 3, wherein the surfactant of the component (B) is polyoxyethylene alkyl ether, an HLB value (NIKKOL) is 16 or greater, and a molecular weight is 1000 or greater or the number of carbons in a hydrophobic group moiety is 16 or greater, and a cloud point is 90°C or higher.

**6.** The cosmetic composition according to any one of claims 1 to 3, wherein the nonionic surfactant of the component (B) is polyglycerol fatty acid ester, the number of repeating units of the glycerin is 6 or greater and the HLB value (NIKKOL) is 12 or greater.

**7.** A skin cosmetic comprising the cosmetic composition described in any one of claims 1, 2, and 4 to 6, and a component (C) cosmetically acceptable medium.

**8.** The skin cosmetic according to claim 7, wherein the component (C) comprises at least a component (c1) UV absorbent.

**9.** The skin cosmetic according to claim 8, wherein the component (c1) comprises at least an oil-soluble UV absorbent and/or a water-soluble UV absorbent.

**10.** The skin cosmetic according to claim 7 or 8, wherein the component (c1) is one type or two or more types selected from the group consisting of 2-ethylhexyl paramethoxycinnamate, 2,4,6-tris[4-(2-ethylhexyloxycarbonyl)anilino]-1,3,5-triazine, 4-tert-butyl-4'-methoxydibenzoylmethane, hexyl ethylaminohydroxybenzoylbenzoate, and phenylbenzimidazole sulfonic acid.

**11.** The skin cosmetic according to any one of claims 8 to 10, wherein a content of the component (c1) is from 0.1 to 25 parts by weight in the cosmetic.

**12.** The skin cosmetic according to any one of claims 7 to 11, wherein the component (C) comprises a component (c2) inorganic powder.

**13.** The skin cosmetic according to claim 12, wherein the component (c2) comprises at least a coloring pigment.

**14.** The skin cosmetic according to claim 12 or 13, wherein the component (c2) comprises a UV protection agent having a particle size of less than 0.3 μm.

**15.** The skin cosmetic according to any one of claims 12 to 14, wherein a content of the component (c2) is from 0.1 to 5 parts by weight per 100 parts by weight of the cosmetic.

**16.** The skin cosmetic according to any one of claims 7 to 15, further comprising a hydrophilic microparticle.

**17.** The skin cosmetic according to any one of claims 7 to 16, the skin cosmetic being an oil-in-water type.

**18.** A film forming agent comprising at least:

(A) 100 parts by weight of at least one vinyl polymer having at least one carbosiloxane dendrimer structure in

a side chain;
(B) from 0.1 to 30 parts by weight of a surfactant; and
(X) water;
wherein an emulsified particle comprising at least the component (A) and the component (B) being is in an aqueous phase; and a weight average molecular weight of the component (A) is at least 80,000.

[FIG. 1]

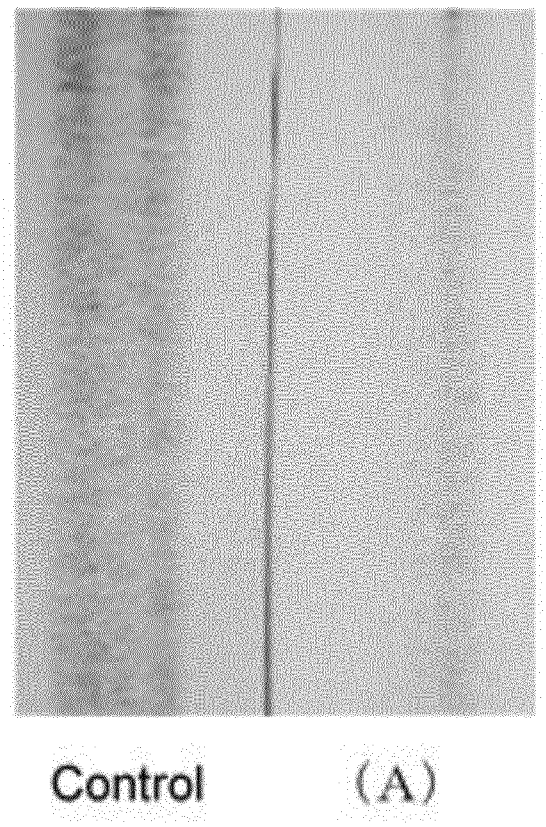

[FIG. 2]

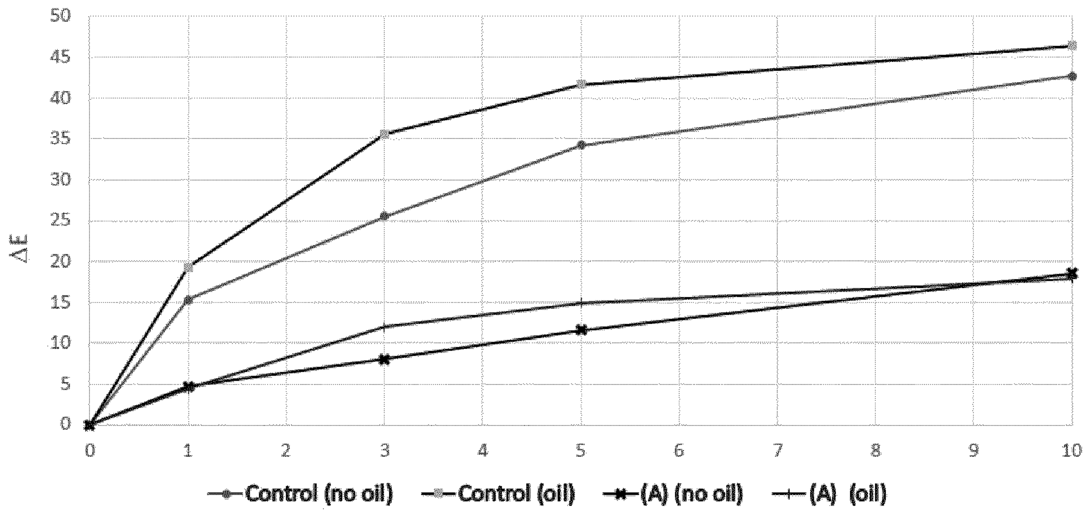
50
45
40
35
30
25
20
15
10
5
0
ΔE
0
1
2
3
4
5
6
7
8
9
10
Control (no oil)
Control (oil)
(A) (no oil)
(A) (oil)

## INTERNATIONAL SEARCH REPORT

International application No. PCT/JP2016/004404

A. CLASSIFICATION OF SUBJECT MATTER
*A61K8/81*(2006.01)i, *A61K8/06*(2006.01)i, *A61K8/37*(2006.01)i, *A61K8/86*(2006.01)i, *A61Q17/04*(2006.01)i

According to International Patent Classification (IPC) or to both national classification and IPC

B. FIELDS SEARCHED

Minimum documentation searched (classification system followed by classification symbols)
A61K8, A61Q17

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2001-192424 A (Dow Corning Toray Silicone Co., Ltd.), 17 July 2001 (17.07.2001), & US 6534590 B1 & EP 1095953 A2 | 1-18 |
| A | JP 2011-149017 A (Dow Corning Toray Co., Ltd.), 04 August 2011 (04.08.2011), & US 2012/0263662 A1 & WO 2011/078407 A1 & EP 2515841 A1 | 1-18 |
| A | JP 2014-40511 A (Dow Corning Toray Co., Ltd.), 06 March 2014 (06.03.2014), & US 2015/0232601 A1 & WO 2014/030770 A2 & EP 2887920 A2 | 1-18 |

☐ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 09 December 2016 (09.12.16) | 20 December 2016 (20.12.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 7309714 A
- JP 2007320960 A
- JP 2014040512 A
- JP 2001019242 A
- JP 11001530 A
- JP 2000063225 A
- JP 2001192424 A


### Non-patent literature cited in the description


- Handbook - cosmetic material/preparation ingredient - revised edition ''Handbook -Cosmetics, Formulation raw materials -Revised. Nikko Chemicals Co., Ltd, 01 February 1977, 854-855
- **BRANDRUP.** J. Polymer Handbook. John Wiley & Sons, Inc, 1999